# EUROPEAN PATENT SPECIFICATION

(11) **EP 3 870 275 B1**
(45) Date of publication and mention of the grant of the patent: **16.07.2025**
(21) Application number: 19877459.8
(22) Date of filing: 23.10.2019
(51) Int. Cl.: A61N 1/36, A61N 1/05, A61B 5/00, A61B 5/24, A61B 5/377

(54) **NEUROSTIMULATION ARTEFACT MINIMISATION**
MINIMIERUNG VON NEUROSTIMULATIONSARTEFAKTEN
MINIMISATION D'ARTEFACT DE NEUROSTIMULATION

(30) Priority: 23.10.2018 AU 2018904012
(43) Date of publication of application: 01.09.2021
(73) Proprietor: Saluda Medical Pty Ltd, Macquarie Park NSW 2113 (AU)
(72) Inventor: SINGLE, Peter Scott Vallack, Artarmon, NSW 2064 (AU); KARANTONIS, Dean, Artarmon, NSW 2064 (AU); SCOTT, Jonathan Brereton, Artarmon, NSW 2064 (AU)
(74) Representative: Abel & Imray LLP
(86) International application number: PCT/AU2019/051160
(87) International publication number: WO 2020/082126

(56) References cited:
- WO-A1-2010/051406
- WO-A1-2017/219096
- WO-A1-2017/219096
- US-A1- 2014 194 772
- US-A1- 2015 282 725
- US-A1- 2016 144 189
- US-A1- 2018 228 547
- BAHMER, A. ET AL.: "Effects of electrical pulse polarity shape on intra cochlear neural responses in humans: Triphasic pulses with cathodic second phase", HEARING RESEARCH, vol. 306, 2013, pages 123 - 130, XP028766244, DOI: 10.1016/j.heares.2013.10.001
- BAHMER, A. ET AL.: "Application of triphasic pulses with adjustable phase amplitude ratio (PAR) for cochlear ECAP recording: I. Amplitude growth function s", JOURNAL OF NEUROSCIENCE METHODS, vol. 205, 2012, pages 202 - 211, XP028891937, DOI: 10.1016/j.jneumeth.2011.12.005

## Description

### Technical Field

The present invention relates to measurement of compound action potentials evoked by a neurostimulator, and in particular to the minimisation of artefact caused by application of an electrical stimulus.

### Background of the Invention

There are a range of situations in which it is desirable to apply neural stimuli in order to give rise to a compound action potential (CAP). For example, neuromodulation is used to treat a variety of disorders including chronic pain, Parkinson's disease, and migraine. A neuromodulation system applies an electrical pulse to tissue in order to generate a therapeutic effect. When used to relieve chronic pain, the electrical pulse is applied to the dorsal column (DC) of the spinal cord, referred to as spinal cord stimulation (SCS). Neuromodulation systems typically comprise an implanted electrical pulse generator, and a power source such as a battery that may be rechargeable by transcutaneous inductive transfer. An electrode array is connected to the pulse generator, and is positioned in the dorsal epidural space above the dorsal column. An electrical pulse applied to the dorsal column by an electrode causes the depolarisation of neurons, and generation of propagating action potentials. The fibres being stimulated in this way inhibit the transmission of pain from that segment in the spinal cord to the brain. To sustain the pain relief effects, stimuli are applied substantially continuously, for example at a frequency in the range of 50-100 Hz.

Neuromodulation may also be used to stimulate efferent fibres, for example to induce motor functions. In general, the electrical stimulus generated in a neuromodulation system triggers a neural action potential which then has either an inhibitory or excitatory effect. Inhibitory effects can be used to modulate an undesired process such as the transmission of pain, or to cause a desired effect such as the contraction of a muscle.

There are a range of circumstances in which it is desirable to obtain an electrical measurement of a compound action potential (CAP) evoked on a neural pathway by an electrical stimulus applied to the neural pathway. However, this can be a difficult task as an observed CAP signal will typically have a maximum amplitude of a few tens of microvolts or less, whereas a stimulus applied to evoke the CAP is typically several volts. Electrode artefact usually results from the stimulus, and manifests as a decaying output of several millivolts or hundreds of microvolts throughout the time that the CAP occurs, presenting a significant obstacle to isolating the much smaller CAP of interest. As the neural response can be contemporaneous with the stimulus and/or the stimulus artefact, CAP measurements present a difficult challenge of implant design. In practice, many non-ideal aspects of a circuit lead to artefact, and as these mostly have a decaying exponential characteristic which can be of either positive or negative polarity, identification and elimination of sources of artefact can be laborious. A number of approaches have been proposed for recording a CAP, including those of King (US Patent No. 5,913,882), Nygard (US Patent No. 5,758,651), Daly (US Patent Application No. 2007/0225767) and the present Applicant (US Patent No. 9,386,934). Other exemplary neurostimulation devices using multipolar stimulation configurations are known from WO 2017/219096 Al, US 2015/282725 Al, US 2014/194772 A1 and US 2018/228547 Al.

Evoked responses are less difficult to detect when they appear later in time than the artefact, or when the signal-to-noise ratio is sufficiently high. The artefact is often restricted to a time of 1 - 2 ms after the stimulus and so, provided the neural response is detected after this time window, data can be obtained. This is the case in surgical monitoring where there are large distances between the stimulating and recording electrodes so that the neural response propagation time from the stimulus site to the recording electrodes exceeds 2 ms. However, to characterize responses evoked by a single implant such as responses from the dorsal columns to SCS, for example, high stimulation currents and close proximity between electrodes are required, and therefore the measurement process must overcome contemporaneous artefact directly, greatly exacerbating the difficulty of neural measurement.

Similar considerations can arise in deep brain stimulation where it can be desirable to stimulate a neural structure and immediately measure the evoked compound action potential produced in that structure before the neural response propagates elsewhere in the brain. Artefact remains a significant obstacle to measurement of neural responses proximal to the stimulus location, with the consequence that most if not all conventional neurostimulation implants, which are necessarily compact devices, do not take any measurements whatsoever of neural responses evoked by the implant's stimuli.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

In this specification, a statement that an element may be "at least one of" a list of options is to be understood that the element may be any one of the listed options, or may be any combination of two or more of the listed options.

### Summary of the Invention

The present invention provides a neurostimulation device according to claim 1, the neurostimulation device comprising:
at least three stimulation electrodes configured to deliver an electrical stimulus in a multipolar fashion to neural tissue; and
at least one measurement electrode configured to record a response of the neural tissue to the stimulus,
wherein the stimulus and a position of the measurement electrode relative to the stimulus electrode are configured such that, in artefact as arising relative to distance from the stimulus electrode, a minima region of the artefact is substantially co-located with the measurement electrode by imposing a mismatch on the respective pairs of stimulation electrodes, in order to thereby co-locate the minima region of the artefact with the measurement electrode , and wherein the device is configured to adaptively alter a stimulation ratio, being a ratio between the respective portions of the stimulus current carried by the stimulus electrodes, depending on which measurement electrode(s) are in use.

The scope of the present invention is defined by the appended claims.

According to an example not covered by the present invention, there is provided an exemplary method of neurostimulation, the method comprising:
delivering an electrical stimulus to neural tissue using at least one stimulation electrode; and
recording a response of the neural tissue to the stimulus using at least one measurement electrode,
wherein the stimulus and a position of the measurement electrode relative to the stimulus electrode are configured such that, in artefact as arising relative to distance from the stimulus electrode, a minima region of the artefact is substantially co-located with the measurement electrode.

The minima region of artefact is defined as a spatial region in which a magnitude of artefact is reduced relative to peak artefact in spatial regions more distal from the stimulation electrode. In some embodiments, the minima region of artefact may comprise a zero crossing region of artefact, being a spatial region in which a magnitude of artefact is reduced relative to peak artefact in spatial regions more distal from the stimulation electrode, and wherein the zero crossing region of artefact contains a zero crossing of artefact. For example, in some embodiments the minima region of artefact may comprise a spatial region in which a magnitude of artefact is less than 75%, more preferably less than 50%, more preferably less than 25%, of a peak artefact arising in spatial regions more distal from the stimulation electrode. Notably, by co-locating the minima region of artefact with the measurement electrode, allows the measurement electrode to be positioned closer to the stimulation site and to thus capture a stronger and less dispersed evoked compound action potential (ECAP), while suffering from less artefact than would occur at even some locations which are further from the stimulation site. In preferred embodiments the stimulus, and a position of the measurement electrode relative to the stimulus electrode, are configured such that a minima of artefact such as the zero crossing of artefact is substantially, or preferably is precisely, co-located with the measurement electrode, so that the measurement electrode experiences negligible artefact relative to peak artefact arising at other distances from the stimulus electrode.

To this end, the present disclosure provides techniques relating to the design of stimulation patterns for spinal cord stimulation and other neuromodulation methods, and provides a set of methods that may be used to reduce or null artefact, thus improving measurement of tissue responses to stimulation such as a neural compound action potential evoked by the applied stimulus.

In the present invention, the stimulus is configured so that the minima region of the artefact is substantially co-located with the measurement electrode, by delivering the stimulus in a multipolar fashion utilising at least three stimulus electrodes, and imposing a mismatch on the respective pairs of stimulation electrodes in order to co-locate the minima region of the artefact with the measurement electrode. For example the stimulus may be delivered in a tripolar fashion by three stimulus electrodes, comprising a central electrode which carries an entire stimulus current and two peripheral electrodes which carry two respective portions of the stimulus current to maintain charge-balanced stimulation, whereby the respective portions of the stimulus current carried by the peripheral stimulus electrodes are mismatched in a manner which causes the minima region of the artefact to be substantially co-located with the measurement electrode.

The desired ratio between the respective portions of the stimulus current carried by the peripheral electrodes, referred to herein as the stimulation ratio, differs depending on which measurement electrode(s) are in use. Accordingly, preferred embodiments provide an artefact minimisation algorithm which delivers a range of stimuli of varying stimulus ratio, at a sub-threshold level which does not recruit a neural response, and observes the artefact caused by each such stimulus at the measurement electrodes, in order to seek a stimulus ratio which minimises artefact observed upon the measurement electrodes in use.

Other embodiments of the first aspect of the invention may employ other stimulus configurations in order to cause the minima region of the artefact to be substantially co-located with the measurement electrode. For example, a quadrupolar stimulation configuration employing four stimulus electrodes, or a multipolar stimulation configuration employing more than four electrodes, may be employed, and may be preconfigured or adaptively configured to deliver stimulus ratio(s) which are mismatched in order to manipulate the location of the minima region of the artefact so that it is substantially co-located with the measurement electrode.

According to an example not covered by the present invention, there is provided an exemplary implantable lead for neurostimulation, the lead comprising a plurality of electrodes, each electrode having an electrode length and an electrode width, and the electrodes being longitudinally spaced apart by an inter-electrode spacing,
wherein the electrode width is greater than the electrode length;
wherein the electrode length is less than 3 mm, and
wherein a ratio of the inter-electrode spacing to the electrode length is between 2 and 3.66.

In embodiments where the lead is a percutaneous lead comprising cuff electrodes passing substantially or wholly around a circumference of the lead, or in the case of non-cylindrical leads around a cross-sectional perimeter of the lead, the electrode width is defined herein as being equal to a width or diameter or largest cross-sectional dimension of the lead.

In embodiments, the electrode length is preferably greater than 1.5mm, for example greater than 1.6mm, more preferably greater than 1.8mm. The electrode length is preferably less than 2.9mm, more preferably less than 2.55mm, more preferably less than 2.2 mm. The electrode length is preferably 2.0mm.

In embodiments, the ratio of the inter-electrode spacing to the electrode length is preferably greater than 2.1, for example being greater than 2.25, more preferably greater than 2.4. The ratio of the inter-electrode spacing to the electrode length is preferably less than 3.5, for example being less than 3.1, more preferably being less than 2.7. The ratio of the inter-electrode spacing to the electrode length is preferably 2.5.

According to an example not covered by the present invention, there is provided an exemplary neurostimulation device comprising:
at least one stimulation electrode configured to deliver an electrical stimulus to neural tissue;
at least one measurement electrode configured to record a response of the neural tissue to the stimulus;
at least one passive electrode proximal to the measurement electrode; and
an impedance connected to the passive electrode, the impedance being configured to reduce artefact arising on the measurement electrode.

According an example not covered by the present invention, there is provided an exemplary method for neurostimulation, the method comprising:
delivering an electrical stimulus to neural tissue using at least one stimulation electrode;
recording a response of the neural tissue to the stimulus using a measurement electrode;
configuring an impedance connected to a passive electrode proximal to the measurement electrode, to reduce artefact arising on the measurement electrode.

The impedance may be preconfigured, and fixed. In alternative embodiments, the impedance may be a variable impedance, and may be adaptively configured by any suitable means in order to reduce, or preferably seek a minima in, artefact observed on the measurement electrode(s). For example the variable impedance may be adaptively configured by use of an artefact minimisation algorithm which delivers a range of stimuli while adjusting the variable impedance to take a range of distinct values, the stimuli being at a sub-threshold level which does not recruit a neural response, and observes the artefact caused by each such stimulus at the measurement electrode(s), in order to seek an impedance value which minimises artefact observed upon the measurement electrode(s) in use at the time. Alternatively, this technique may adopt the use of supra-threshold stimuli which do recruit neural responses, and may measure the total energy of the neural response + artefact, and may adjust the variable impedance in such a way as to seek a reduction or minima of such energy. The artefact minimisation algorithm of such embodiments may operate simultaneously with the above-described artefact minimisation algorithm of some embodiments of the first and second aspects of the invention, for example by use of two simultaneously or contemporaneously running feedback loops.

The impedance may comprise a resistance, or a reactance. The impedance may be variable by being implemented in the form of a switched capacitor resistance, configured to present a controllable resistance as defined electronically by a switching rate or pulse width modulation of the switched capacitor.

In some embodiments, the impedance is connected between a pair of passive electrodes positioned either side of the measurement electrode. In such embodiments, where more than one measurement electrode is used, one of the passive electrodes is preferably positioned between the measurement electrodes, and the other of the passive electrodes is preferably positioned between the stimulus electrode(s) and the measurement electrodes. Thereby, a pair of electrodes not involved in the stimulation or measurement can be used to steer a minima or zero of artefact onto a measurement electrode.

According to an example not covered by the present invention, there is provided an exemplary neurostimulation device comprising:
at least one stimulation electrode configured to deliver an electrical stimulus to neural tissue; and
at least one measurement electrode configured to record a response of the neural tissue to the stimulus;
wherein the electrodes are arranged in a longitudinal array and at least one of the electrodes is configured to exhibit a greater resistance in a longitudinal direction as compared to a resistance of that electrode in a transverse direction.

In some embodiments, an electrode may be configured to exhibit a greater resistance in a longitudinal direction by providing the electrode with transverse slots or ribs, configured to increase a longitudinal current path length.

According to an example not covered by the present invention, there is provided an exemplary neurostimulation device comprising:
at least one stimulation electrode configured to deliver an electrical stimulus to neural tissue;
a current source configured to produce the electrical stimulus; and
at least one measurement electrode configured to record a response of the neural tissue to the stimulus;
wherein the stimulation electrode is split into at least two electrode portions configured to create a discontinuity in a tissue-electrode interface, and wherein the current source is connected to the electrode portions by respective resistors.

In some embodiments, a first resistance connecting the current source to a first portion of the split electrode is different from a second resistance connecting the current source to a second portion of the split electrode. The first and second resistances are preferably selected, or are adaptively controlled, in a manner to counteract asymmetric voltages upon an electrode-tissue interface.

### Brief Description of the Drawings

An example of the invention will now be described with reference to the accompanying drawings, in which:
Fig. 1 schematically illustrates an implanted spinal cord stimulator;
Fig. 2 is a block diagram of the implanted neurostimulator;
Fig. 3 is a schematic illustrating interaction of the implanted stimulator with a nerve;
Fig. 4 illustrates an implantable lead electrode array;
Fig. 5 is an equivalent circuit of a single cuff electrode;
Fig. 6 is a plot of predicted artefact amplitude as a function of the number of sections into which an electrode cuff is nominally split in a simulation;
Fig. 7 pictorially depicts a stimulation and measurement configuration of an electrode lead;
Fig. 8 is a plot of the stimulus voltage and resultant saline artefact;
Fig. 9 is a simplified circuit diagram showing simulated slicing of four electrodes into two slices each, and interaction thereof with a mesh resistance representation of saline;
Fig. 10 is a plot of the simulated potential arising on interface layers of electrode four as a function of nominal slice position, in response to a stimulus applied between electrodes two and one, in a 21 slice simulation, both at the end of the stimulus and after 1ms relaxation;
Fig. 11 is a plot of the simulated potential arising on the stimulus electrode at the end of the biphasic stimulus, in a 21 slice simulation,
Fig. 12 is a plot of artefact, both measured and simulated, as a function of stimulus current;
Fig. 13 is a plot of artefact, both measured and predicted, measured between adjacent electrode pairs, as a function of displacement from the stimulus electrodes;
Figs. 14a and 14b are diagrams of current flow paths in two stimulating electrodes and one passive electrode, during and after a stimulation pulse respectively;
Fig. 15 is a simulated plot of electric field in saline surrounding two stimulating and two passive electrodes shortly after completion of a stimulus;
Fig. 16 is a simulated plot of electric field in saline surrounding two stimulating and one passive electrode shortly after completion of a stimulus;
Figure 17 shows a method of tripolar stimulation for artefact minimisation;
Figure 18 illustrates decomposition of tripolar stimulation into bipolar stimuli;
Figure 19 illustrates artefact summation from the components of Fig. 18;
Figure 20 shows simulated artefact for tripolar stimulation as the term A is varied;
Figure 21 shows a variable tripolar stimulation waveform in accordance with one embodiment of the invention;
Figure 22 shows artefact amplitude on electrodes E4-E8 as the tripolar stimulus ratio is varied;
Figures 23a-23d illustrate a feedback control loop for automatically adjusting multipolar stimulation ratios to seek a minima of artefact, in accordance with an embodiment of the invention;
Figure 24 illustrates another embodiment, in which a birding resistor minimises artefact;
Figure 25 illustrates a 4-contact epidural electrode array having conventionally sized electrodes, and an epidural electrode array in accordance with an embodiment of the present invention, comprising shortened electrodes;
Figure 26 shows a circuit model of a single electrode in a saline bath;
Figure 27 shows the voltage along the surface of the electrode of Fig. 26;
Figure 28 shows a circuit model of a pair of stimulating electrodes in a saline bath;
Figure 29 shows the voltage along the surface of the electrodes of Fig. 28;
Figure 30 shows a circuit model of a pair of recording electrodes in a saline bath;
Figure 31 shows the voltage along the surface of the electrodes of Fig. 30;
Figure 32 shows a compression algorithm used to highlight artefact in Fig 34;
Figure 33 shows a stimulation waveform;
Figure 34 shows the compressed field in a saline bath in response to a biphasic stimulation;
Figure 35 shows the uncompressed voltages along the electrode surface;
Figure 36 illustrates a paddle electrode array having shortened electrodes;
Figure 37 illustrates a neurostimulation lead having split electrodes;
Figure 38 illustrates another neurostimulation lead having split electrodes in accordance with another exemplary configuration; and
Figure 39 illustrates a neurostimulation electrode having increased longitudinal resistance.

### Description of the Preferred Embodiments

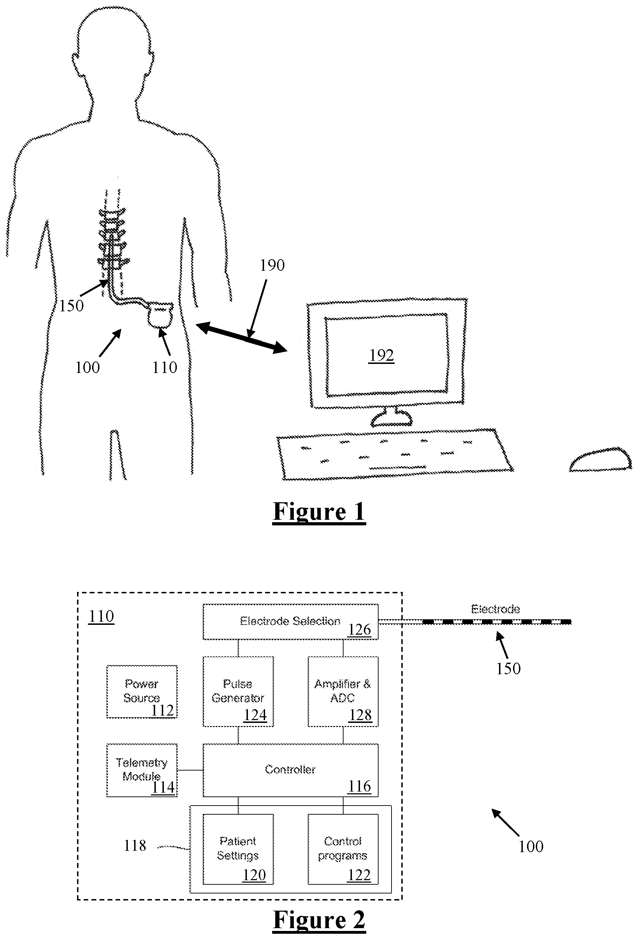

**Fig. 1** schematically illustrates an implanted spinal cord stimulator 100. Stimulator 100 comprises an electronics module 110 implanted at a suitable location in the patient's lower abdominal area or posterior superior gluteal region, and an electrode assembly 150 implanted within the epidural space and connected to the module 110 by a suitable lead. Numerous aspects of operation of implanted neural device 100 are reconfigurable by an external control device 192. Moreover, implanted neural device 100 serves a data gathering role, with gathered data being communicated to external device 192 via any suitable transcutaneous communications channel 190.

**Fig. 2** is a block diagram of the implanted neurostimulator 100. Module 110 contains a battery 112 and a telemetry module 114. In the present disclosure, any suitable type of transcutaneous communication 190, such as infrared (IR), electromagnetic, capacitive and inductive transfer, may be used by telemetry module 114 to transfer power and/or data between an external device 192 and the electronics module 110. Module controller 116 has an associated memory 118 storing patient settings 120, control programs 122 and the like. Controller 116 controls a pulse generator 124 to generate stimuli in the form of current pulses in accordance with the patient settings 120 and control programs 122. Electrode selection module 126 switches the generated pulses to the appropriate electrode(s) of electrode array 150, for delivery of the current pulse to the tissue surrounding the selected electrode(s). Measurement circuitry 128 is configured to capture measurements of neural responses sensed at sense electrode(s) of the electrode array as selected by electrode selection module 126.

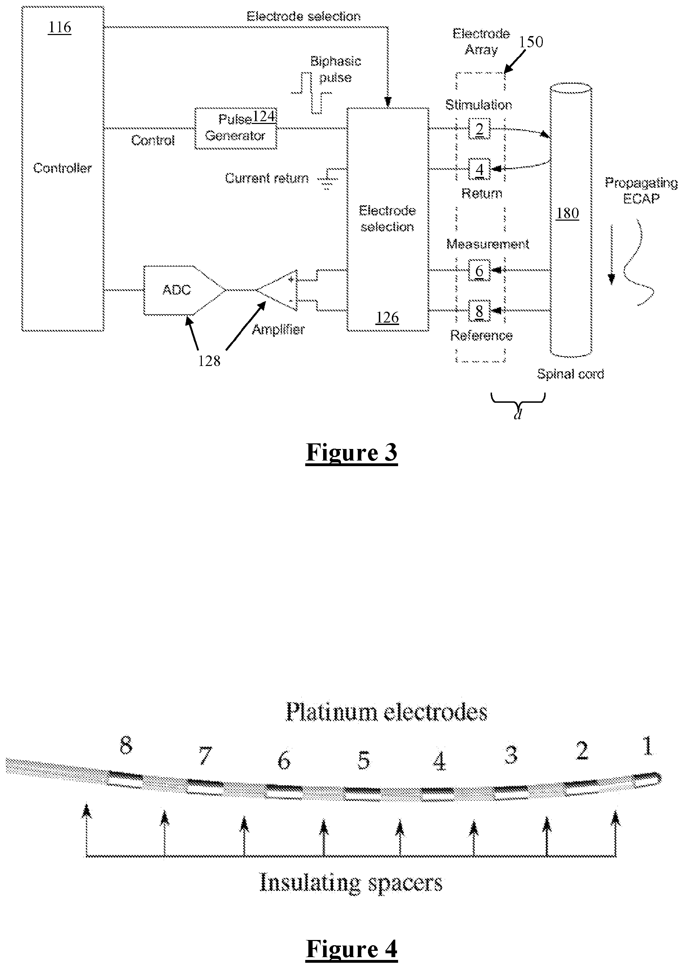

**Fig. 3** is a schematic illustrating interaction of the implanted stimulator 100 with a nerve 180, in this case the spinal cord however alternative embodiments may be positioned adjacent any desired neural tissue including a peripheral nerve, visceral nerve, parasympathetic nerve or a brain structure. Electrode selection module 126 selects a stimulation electrode 2 of electrode array 150 to deliver an electrical current pulse to surrounding tissue including nerve 180, and also selects a return electrode 4 of the array 150 for stimulus current recovery to maintain a zero net charge transfer.

Delivery of an appropriate stimulus to the nerve 180 evokes a neural response comprising a compound action potential which will propagate along the nerve 180 as illustrated, for therapeutic purposes which in the case of a spinal cord stimulator for chronic pain might be to create paraesthesia at a desired location. To this end the stimulus electrodes are used to deliver stimuli at any therapeutically suitable frequency, for example 30 Hz, although other frequencies may be used including as high as the kHz range, and/or stimuli may be delivered in a non-periodic manner such as in bursts, or sporadically, as appropriate for the patient. To fit the device, a clinician applies stimuli of various configurations which seek to produce a sensation that is experienced by the user as a paraesthesia. When a stimulus configuration is found which evokes paraesthesia, which is in a location and of a size which is congruent with the area of the user's body affected by pain, the clinician nominates that configuration for ongoing use.

The device 100 is further configured to sense the existence and intensity of compound action potentials (CAPs) propagating along nerve 180, whether such CAPs are evoked by the stimulus from electrodes 2 and 4, or otherwise evoked. To this end, any electrodes of the array 150 may be selected by the electrode selection module 126 to serve as measurement electrode 6 and measurement reference electrode 8. Signals sensed by the measurement electrodes 6 and 8 are passed to measurement circuitry 128, which for example may operate in accordance with the teachings of International Patent Application Publication No. WO2012155183 by the present applicant. Nevertheless, artefact remains a significant obstacle to measurement of neural responses proximal to the stimulus location. The present disclosure first investigates the artefact phenomenon in more detail, and then provides a number of novel solutions based on the findings.

The current pulses delivered through platinum electrodes by medical implants to recruit neurones give rise to slowly-decaying voltage tails, called "artefact". These tails make measurement of evoked potentials following the pulses very difficult. We present evidence to show that in a typical clinical scenario these tails are mostly caused by concentration gradients of species induced in the electrical double layer adsorbed onto the platinum surface of both stimulating and passive electrodes. A compact model is presented that allows simulation of these artefacts. This model can be expected to prove useful in predicting the effectiveness of techniques to reduce the artefact amplitude.

Considerable interest has arisen recently in sensing neural activity in the presence of, and quickly following, neural stimulus pulses in order to apply feedback in neuromodulation systems. The evoked compound action potentials (ECAP) synchronised with the stimulus and measured near the stimulation site betray the extent of neural recruitment resulting from the pulse. Measured ECAP thus can be of great assistance both during the implantation surgery and for automatic adjustment of the stimulus pulse amplitude and locale in routine use.

A typical neuromodulation pulse has an amplitude of several volts, whereas the amplitude of the signal visible at an electrode as a result of a nerve firing may be only a few microvolts. To be useful, the evoked responses must be recorded starting within about a hundred microseconds of the end of the stimulus pulse. To make the job of the recording electronics even more difficult, the artefact tails have amplitudes between millivolts and tens of microvolts depending upon the spacing between the stimulus and recording electrodes. Sensor electrodes which are closest to the stimulation site are best positioned to assess recruitment, but have greater artefact, as ECAP disperses as it propagates from the recruitment site. It is demanding to design amplifiers capable of processing these signals for analog-to-digital conversion, particularly in CMOS.

In this light, a compact model of the impedance of the electrode-electrolyte interface is presented. This model uses Constant-Phase Elements (CPEs) amongst other more common elements in an equivalent-circuit representation of the interface between electrode and electrolyte. The model is able to accurately represent the impedance of the interface. **In** a circuit simulator the model allows prediction of the residual voltage and artefact tails in some circumstances, but not all. As will be seen below, the model predicts zero artefact in certain circuit configurations, whereas measurement reveals that significant artefact actually arises in such configurations. This makes clear that there is a mechanism at work that is not captured by the single-CPE compact model.

Accordingly, the present inventors introduce a split-electrode model. It has been hypothesized that artefact can arise not only through residual charge left in the double layer surrounding an electrode after it conducts a current, but also by virtue of an uneven distribution of that charge along the surface of an electrode. Such an uneven charge distribution can arise even without any net current flowing through the electrode into the electronics. One might visualise counter-charges in the diffuse region of the electrical double layer piling up at one end of an electrode as a result of surface conduction, resulting from fields generated by the stimulating electrodes. Although for many purposes a more elaborate model is necessary, this idea of charges piling up at one end of an electrode suggests a straightforward way that a compact model might be constructed to simulate the phenomenon.

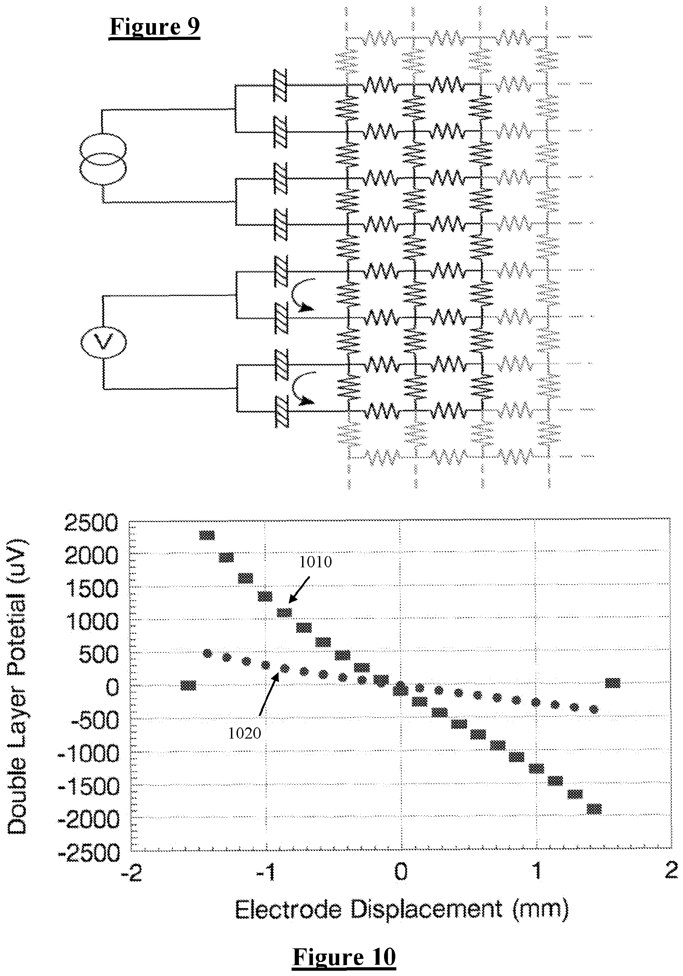

In the proposed split-electrode model, the electrode is split into multiple sections or "slices" that are modelled separately. The single-branch model of an electrode is replaced with one having n branches, each contributing 1/n times the total admittance (area) presented by the original electrode. The branches are joined together at a single node at the metal side of the electrode, but are connected only through the resistor mesh representing the bulk fluid on the fluid side of the interface (as shown in Fig. 9, discussed further in the following).

A rotationally-symmetric situation is considered, so that simulation is straightforward using a two-dimensional representation, and comparative measurements can be made with a cylindrical, implantable, platinum electrode array such as an octrode (eight electrode array) or dodecatrode (twelve electrode array). A typical implantable lead electrode array with a set of 8 electrodes (cylindrical platinum cuffs), suitable for use as array 150 in the embodiment of Figures 1-3, is pictured in **Fig. 4****.** Each cuff is about 1.3mm in diameter and 3mm long, spaced by 4mm insulating sections.

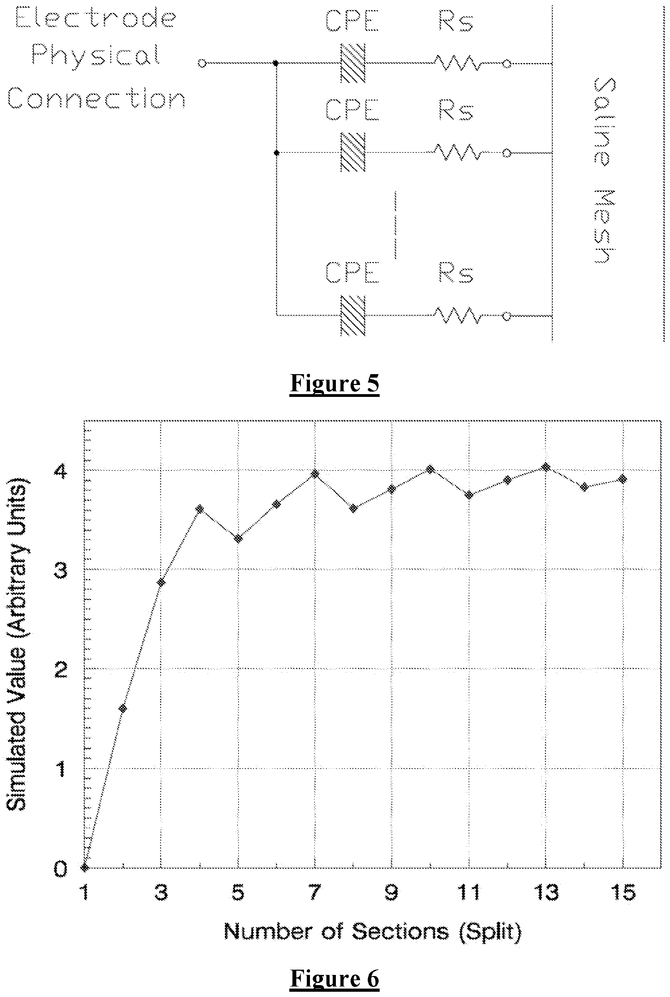

The equivalent circuit of a single cuff, omitting the diodes and memristors for clarity, is shown in **Fig. 5****.** As can be seen in Figure 5, the equivalent circuit of a single 3mm cuff of the electrode array becomes a sequence of n parts each with its own CPE, and each tapping into different geometrical points in the mesh of resistors representing the electrolyte. While diodes must be included in such models except for small-signal simulations, such diodes are omitted from the present Figures for clarity. For small-signal simulations of scenarios that are safe for use in humans, the diode-memristor branch elements can be ignored, however the diodes at least must be included to detect and model nonlinear Faradaic effects. Base model parameters for the CPE used in the circuit were obtained, trimmed to show agreement with the impedance observed looking into electrodes 1 and 2, and scaled according to the split factor in use. Our simulations use base values of m = 1.5, |*Z*| = 6500Ω at 1 Hz, and R_{S} = 12 Ω. The SPICE equivalent network was generated using a density of k = 1.3 from 10 mHz to 500 kHz.

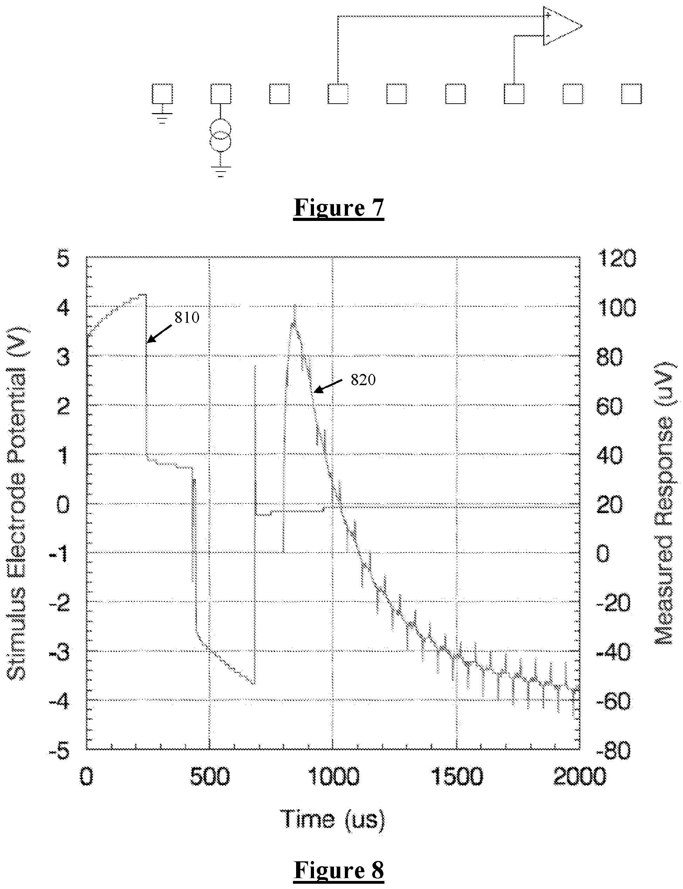

Resistor mesh values were calculated using a measured value of saline conductivity of 6400 mm and on a grid selected to suit the split factor. In this description we will chiefly consider one particular circuit arrangement, as depicted pictorially in Fig. 7. Stimulation current is applied to the second cylindrical cuff of the lead, marked as electrode 2 in Figures 4 and 7. The stimulation current return will be via the first cuff, electrode 1. The voltage of interest is that appearing between the fourth and seventh cuffs, electrodes 4 and 7. This represents a typical, clinically-desirable arrangement for use in a feedback implant. The stimulus pulse will be a so-called biphasic pulse consisting of +5 mA delivered for 240 µs, zero current for 200 µs, -5mA delivered for 240 µs, and finally disconnection of the drive electronics. The form of the stimulus pulse is not particularly germane to the issue of how artefact arises so that the solutions presented in the following can also be applied to other stimulation regimes.

This configuration was simulated using a single-CPE model for the electrodes in 0.1x Phosphate Buffered Saline (PBS). In the case of a single-branch electrode model, absolutely zero artefact is predicted to appear between electrodes 4 and 7, assuming there is no load presented by the electronics. However, when the electrode model is split into ever-greater numbers of slices, the prediction changes. **Fig. 6** dramatically shows the impact. Fig. 6 shows the predicted artefact amplitude as a function of the number of sections into which an electrode cuff is split, in the SPICE simulation. Fig. 6 would suggest that splitting the electrodes into 7 or more slices in the simulation will be required to give reasonable accuracy of artefact amplitude. Accordingly, a split factor of 11 will be used in the following, except where noted. Since the resistor mesh representing the electrolyte (shown in Fig. 9) is also split into slices of the same geometric size, say 3/11ths of a millimetre or finer, the number of mesh nodes involved to simulate an electrode extending over 60mm of electrolyte can get quite large. SPICE takes from a few seconds to a few minutes to run each such simulation on a typical personal computer.

Comparison with measurement was performed. A Saluda Medical Pty Ltd "Evoke" implant was used to generate pulses and amplify the measured signals. The wiring arrangement is depicted pictorially in **Fig. 7****.** Stimulus is shown delivered to electrode 2 with ground return via electrode 1. Response is measured at electrode 4 with respect to electrode 7. The 0.1x PBS was created using Medicago 09-2051-100 PBS tablets and de-ionised water at the rate of 1 tablet per litre. This is a common phantom for tissue in cerebrospinal fluid (CSF). The received signal is digitised by the implant, but was also digitised using a Tektronix TPS2014 isolated-input oscilloscope from a test port provided by the implant IC. The same data can be obtained from the implant, but only at a lower sample rate, approximately 16k samples per second. There is an uncertain additive contribution from common mode signals owing to the implant amplifier's finite Common-Mode Rejection Ratio (CMRR), which is no worse than 75dB.

**Fig. 8** shows the stimulus voltage 810 (left axis) and a typical measured response signal 820 (right axis) for a 5 mA pulse, applied from the second to the first electrode, and measuring the voltage present between the fourth and seventh electrodes, as depicted in figure 7. The response 820 measured (in µV, right axis) between the fourth and seventh electrodes is blanked until about 800 µs after the start of the stimulus 810 (depicted in V, left axis). Response 820 is a manifestation of the artefact which obscures neural measurements *in vivo,* and it is to be noted that the dynamic range of artefact 820 is around 150 uV which is considerably larger than the voltages observed with evoked neural responses, illustrating the severity of the problem posed by such artefact. The series of small impulses on the received pulse tail 820 are caused by the implant ADC operation and are not part of the signal. As can be observed in figure 8, the front-end amplifier (FEA) used to record signal 820 is blanked during the pulse 810, and the blanking released about 100 µs after the stimulus current switches off. An arbitrary dc offset has been subtracted from the test port voltage as the Implant Pulse Generator (IPG) FEA is ac coupled. The array of small spikes spaced about 31 µs apart are clock feedthrough associated with the analog-to-digital conversion inside the implant chip and should be ignored. Switching spikes are visible on the stimulus voltage trace 810 in figure 8. These cause a contribution to the artefact that does not vary with stimulus magnitude. As will be seen below this contributes a constant offset to the magnitude of the measured artefact.

It proves difficult to obtain agreement between simulation and measurement of artefact such as that observed at 820 in Figure 8. This difficulty is now attributed by the present inventors to artefact being the sum of several components that to a large degree cancel each other out. Thus small deviations in any one component, associated perhaps with a particular cuff (contact, also referred to herein as an electrode), can give rise to relatively large variation in final artefact value. We believe this accounts for the anecdotal observation that observed artefact can be better or worse on different contacts, for no obvious reason, even in saline where the inhomogeneity of tissue is not an issue. Our model allows these components to be identified separately, as follows.

A first such artefact component is artefact from passive electrodes. All the electrodes on a lead are exposed to a voltage gradient along their length during the pulse. Charge accumulates at one end of each conductive cuff compared to the other. There is generated a voltage gradient in the tissue or electrolyte in which the lead is immersed by the dipole of the stimulating and return electrodes, being electrodes 2 and 1 in this example. Charge is more easily displaced along the surface of the cuff than the surrounding medium. Once the pulse is over, even an electrode that was not electrically connected, and which conducted zero net current, acquires a charge imbalance along its surface that manifests itself as a transient net potential difference between the metal of the cuff and the bulk medium.

**Fig. 9** shows a simplified circuit with 4 electrodes, each electrode being split into merely two slices. While the simulations herein used 11 slices, this two slice simplified circuit is provided to assist in visualising the generation of artefact in passive electrodes. Arrows in Figure 9 show current induced by the stimulus in one end of a passive electrode and out of the other end of the passive electrode. The present inventors recognise that the CPEs of each such single electrode become charged by the "circulating current".

**Fig. 10** plots the simulated potential resulting from accumulated charge in the interface layers as a function of position along electrode 4, purely as a result of the 5 mA stimulus current flowing between electrodes 2 and 1. The electrode was split into 21 slices for this simulation. Rectangular symbols 1010 indicate the simulated voltage recorded at the end of the biphasic stimulus pulse depicted in figure 8. The dots 1020 represent the simulated data at 1ms elapsed time, showing the relaxation. The electrode was not connected, so net electrode current remains zero throughout. It is noted that the distribution is not symmetrical along the length of the electrode, and for example the slice potential 1010 at the displacement -1.4mm is about 2.3 mV whereas the slice potential 1010 at the displacement +1.4mm is about -1.9 mV. The simulation suggests that the average or net potential is around 100 µV across electrode 4 from contact to the bulk saline. The residual potential is larger on individual electrodes that are closer to the stimulating pair and smaller on ones further away. This distribution relaxes at the end of the pulse as the charge redistributes.

The same mechanism operates on electrode 7, but less than 2 µV results, as compared to the net 100 µV on electrode 4. Thus, a total of ≈ 100 µV contribution is made to V(4,7), being the sensed voltage, chiefly because of the net voltage between the tissue side and the metallic side of electrode 4. The first artefact component, being artefact from passive electrodes, is thus a considerable contributor to the artefact problem.

A second artefact component is artefact from Stimulation Electrodes. The stimulating electrode, number 2 in our example, develops around 25 times the charge gradient along the length of its surface compared with electrode 4, since one end of it is much closer to the ground return path provided by the adjacent electrode 1.

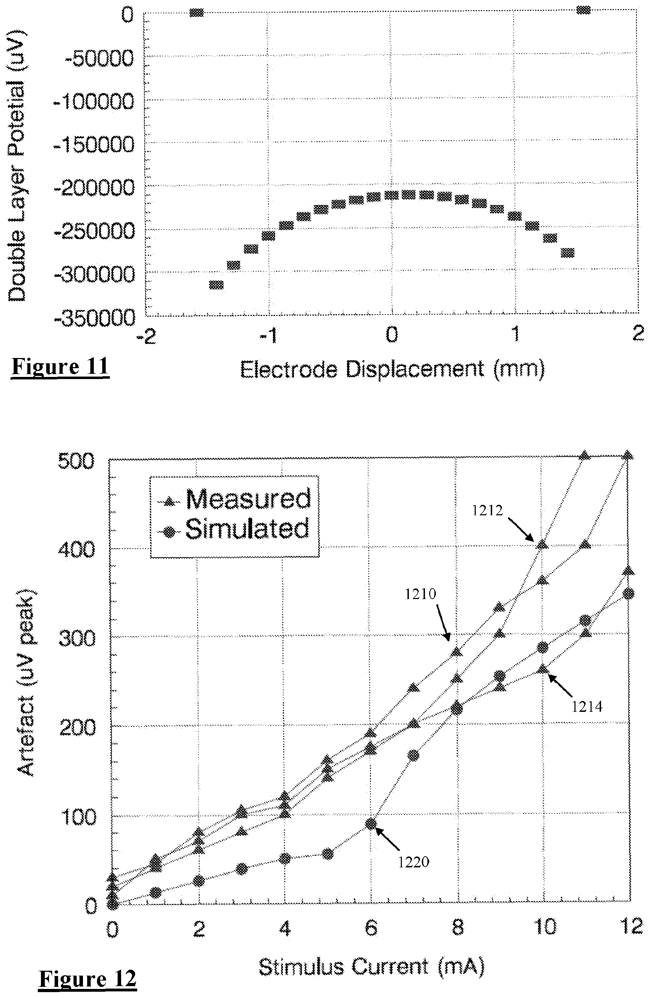

**Fig. 11** shows the simulated potential resulting from accumulated charge in the interface layers as a function of position along electrode 2 as a result of the 5mA stimulus pulses. The simulation data shown in Fig. 11 is at the end of the biphasic stimulus pulse 810 depicted in figure 8. The y-axis units are chosen to be the same as those in figure 10. At the edge of this electrode, even a stimulus pulse of 5 mA causes nonlinear effects, although much less nonlinear action would be expected if the current were to be delivered evenly across the area of the cuff. This large charge gradient relaxes at the end of the pulse, inducing potential differences between all the electrodes along the lead. The contribution to the measured voltage V(4,7) is ≈ 100 µV. It is further noted that the average offset from zero represents the charge accumulated after the stimulus. The variation of potential along the electrode shows the charge is accumulated unevenly. The charge or species concentration in the double layer is not the same at each point along the electrode.

The ground return electrode 1 responds in the same manner as the stimulating electrode 2, but the polarity of its contribution is the reverse, and it is a different distance from the receiving pair 4,7. The ground return electrode 1 thus also produces an artefact contribution to the measured voltage V(4,7). The difference between the contributions from the stimulating and ground electrodes is typically ≈ 50 µV, for a 5 mA stimulus. The second artefact component, being artefact from stimulation electrodes, is thus also a considerable contributor to the artefact problem.

A third artefact component is Common-Mode Artefact. The total charge accumulated across each of the stimulating and return electrodes between the metal and the medium (as different from the difference along the cuff purely on the electrolyte side) is many hundreds of mV, as seen in figure 11. The receiving amplifiers in state of the art Saluda Medical Evoke implants typically have a common mode rejection ratio (CMRR) in the range of 80dB. The circuit ground remains connected to the return current electrode. The average voltage across the ground electrode appears as a common-mode signal. This gives rise to ≈ 50 µV of additional artefact. The third artefact component, being common mode artefact, is thus also a considerable contributor to the artefact problem.

Noting these components of artefact, it is possible to address the question of what total artefact is generated by these mechanisms when they are operating together. **Fig. 12** shows artefact as a function of the peak stimulus current, both measured and simulated. The three measured traces 1210, 1212, 1214 represent the normal variation between supposedly-identical leads. Note the zero-offset on measured traces resulting from common-mode and switching signals. The difference in magnitudes between the simulated trace 1220 and the measured traces is attributed to the ideal nature of the compact model, within which all electrodes are identical and common-mode signals have no contribution. In the circumstances, agreement is considered to be excellent.

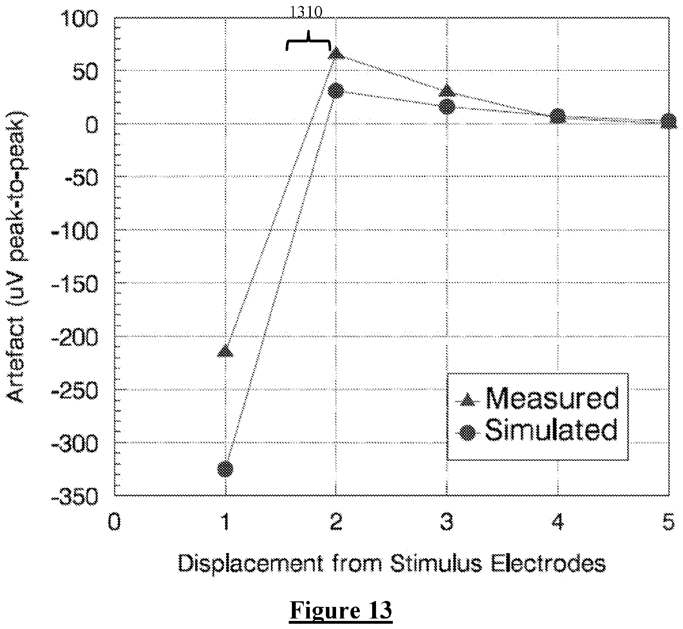

**Fig. 13** presents measurement and prediction of the artefact measured between adjacent electrode pairs v(3,4), v(4,5), v(5,6), v(6,7), and v(7,8), indicated on the x-axis by their respective displacement in number of electrodes from the stimulus electrodes. An offset at zero stimulus current of about 20 µV, as observed in Figure 12, has been subtracted from measured data shown in Figure 13. As described in the preceding, a stimulus of 5mA is applied at electrode 2 with ground return through electrode 1. The simulation predicts the paradoxical change in sign of the artefact between the first electrode pair (v(3,4), displacement of 1) and second electrode pair (v(4,5), displacement of 2). This change in sign of artefact has previously been observed but appeared paradoxical and added considerable intractability to the problem of solving artefact. That this change in sign is predicted by the present model lends considerable confidence to the veracity of the model. Moreover, this indicates that there exists a zero-crossing of artefact, or more generally a zero crossing region indicated by 1310, which presents an opportunity for low-artefact neural measurement, an observation exploited by the presented embodiments of the present invention.

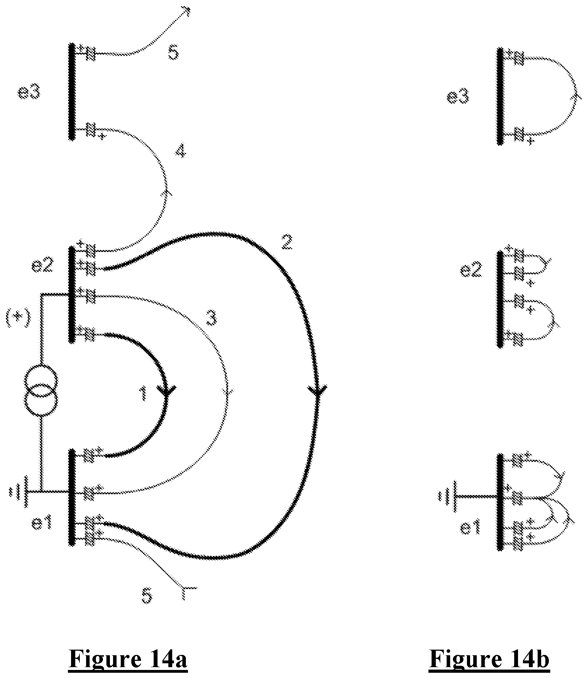

A fourth artefact component is the contribution from a single passive cuff electrode. The impact of charges "passively displaced" by flowing stimulus currents is key to understanding, and cannot be underestimated. To emphasise this point and aid understanding, consider **Fig. 14****,** which is a diagram of current flow paths in the simplified case of two stimulating and one passive electrode, during (Figure 14a) and then after (Figure 14b) a stimulation pulse. The current labelled "5" may be 100 times smaller than currents "1", "2", and "3", but it leaves e3 with significant displaced charge. This diagram suggests how a completely passive metal structure becomes "charged", that is how it comes to host a displaced population of mobile species in the Gouy-Stern-Chapman layer.

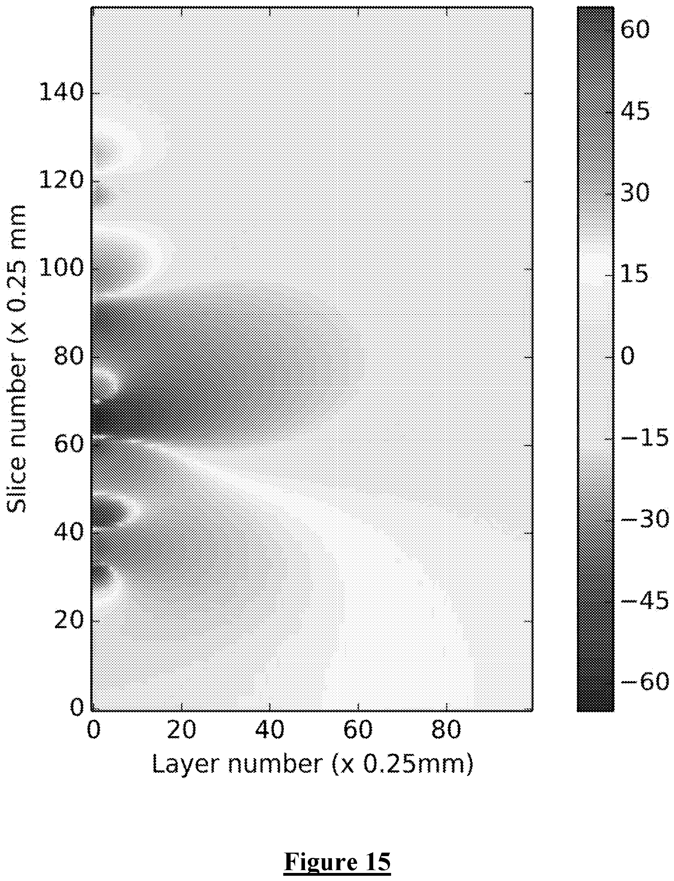

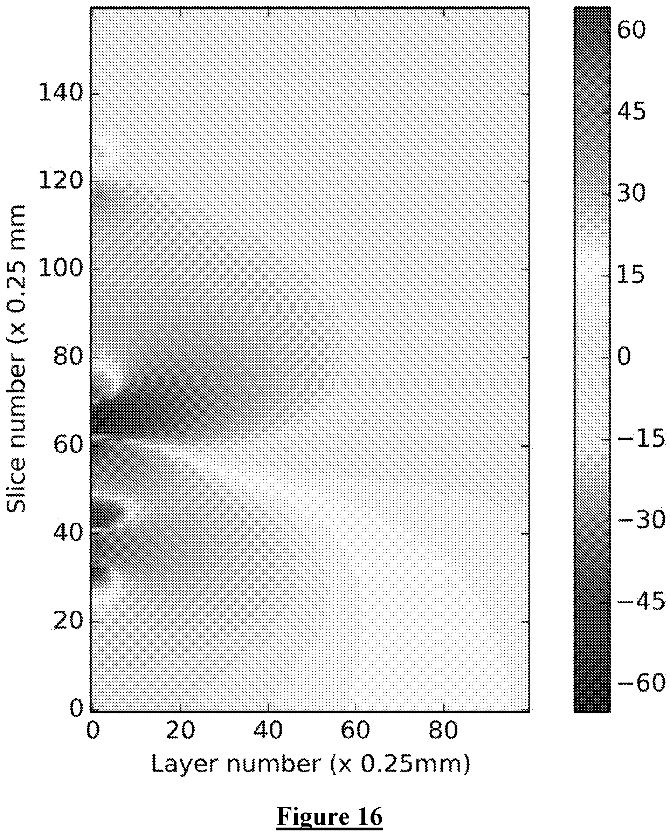

Figs. 15 and 16 then show the impact on residual field of including or omitting a single passive cuff. **Fig. 15** is a plot of electric field in the saline surrounding a set of electrodes shortly after removal (completion) of a stimulus, for two stimulating and two passive electrodes. The electrodes are placed along the y-axis. The electrodes are completely disconnected, so that no current flows into or out of the electrolyte. **Fig. 16** is a plot of electric field in the saline surrounding a set of electrodes shortly after removal of stimulus, for two stimulating and one passive electrodes; in comparison with figure 15 the only change in Fig. 16 is that one of the passive electrodes (e3) has been removed. Comparison of these two figures quickly reveals that an added piece of passive disconnected metal (in Fig. 15) introduces new regions (around slice 100 in the vicinity of electrode e3) where the field changes polarity, passing through zero, where there was before (in Fig. 16) no zero at all. Of course, this phenomenon will occur with any metallic structure in proximity of the stimulating electrodes, not only components of the implanted lead itself.

The observation that this electrode model only predicts artefact at all when the electrode is modelled as a parallel series of "slices" that are free to accumulate unequal charges confirms the conjecture that uneven charge distribution on the surface of individual electrodes contributes to the long pulse tails referred to as "artefact" observed on implanted measurement electrodes. It is further noted that the model shows that surface charge imbalance is the main or possibly sole contributor to artefact, as Fig. 12 demonstrates that the model can account for the magnitude of artefact observed in a well-designed implant with good common mode rejection ratio in the front end amplifier used for recording.

The preceding analysis thus provides confirmation of an electrode-intrinsic mechanism responsible for electrode artefact. By "electrode-intrinsic" we mean a phenomenon that is an inescapable physical consequence of the electrode design, inherent to the geometry and materials of the electrode, and independent of electrical action, connection, or loading of associated electronics. While electrical efforts to minimise artefact tails in neuromodulation systems arising from electrical action, connection, or loading of associated electronics are important and ongoing, it is clear that the artefact measured in the present description inescapably arises within the electrode-electrolyte system itself. Thus, even a perfect front end amplifier will encounter this signal.

Armed with the new understanding that surface charge equilibrium will set a lower (best case) limit on the artefact which will necessarily be added to (i.e. arise contemporaneously with, and obscure) an evoked compound action potential, it becomes possible and important to consider how electrodes might be designed to intrinsically minimise the phenomenon, and also to consider what electrical steps might be taken to accommodate or even beneficially exploit this electrode-intrinsic mechanism and the findings of the preceding analysis. The following solutions are proposed.

Specifically, we describe several techniques conceived on the basis of the findings of the preceding analysis. These techniques include tripolar stimulation, bridged electrodes, and changes to electrode size and shape.

Referring again to Figure 13, it is noted that the sign of the artefact changes as position changes along the lead with increasing distance from the stimulation electrode. In particular, when measuring V(3,4) at a displacement of 1 electrode, the artefact is negative, but when measuring V(4,5) at a displacement of 2 electrodes from the stimulation electrode, and at greater displacements, artefact is positive. This is confirmed both by simulation and by measurement, as indicated in the graph of Figure 13. While not shown in Figure 13, other measurement electrode configurations, such as measuring V(3,5) or V(3,6) can also experience such a change in sign of artefact with changing displacement from the stimulation site.

The present invention recognises that this change in sign of artefact with increasing displacement from the stimulation electrode suggests that there exists at least one location along the lead where the sum of the artefact components is zero. We therefore turn to consider ways in which the artefact may be minimized or substantially eliminated at the location of the measurement electrode, even if artefact is non-zero at locations away from the measurement electrode, by causing the location of a zero-crossing of artefact to be substantially co-located with a measurement electrode. We propose that this may be effected in one or more of a number of ways, including by means of changing the layout of electrodes, changing the interconnection of the electrodes, and/or changing the spatio-temporal distribution of stimulus current delivered.

The present disclosure recognises that artefact zero can be steered with careful tripolar stimulation. In particular, the present inventors recognise that the artefact zero can be displaced onto the 4th electrode by adding some contributory current to the 3rd electrode, so that the 2nd and 3rd electrodes combined provide the stimulus current that is returned via the 1st electrode. In general, the principle is to stimulate through three or more electrodes in some fashion, often utilising an asymmetric current division, in a manner that minimises artefact on another electrode or electrodes.

In saline, simulation predicts that there will be a zero of artefact on electrode 4 when stimulus current X flowing into electrode 1 is fed by yX out of electrode 2 and (1-y)X is fed out of electrode 3. Measurement puts these currents close to 0.5X and 0.5X respectively, y=0.5.

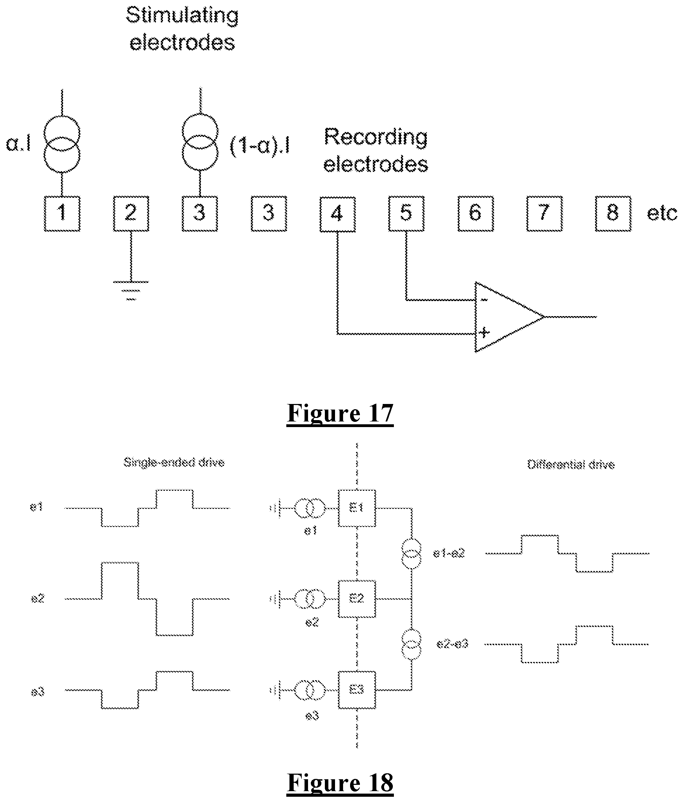

**Figure 17** shows a method of tripolar stimulation where the current to the outer-contacts can be varied using a parameter alpha. A concise terminology is required to describe general methods of dividing current between multiple electrodes: the term [E,e,A] is used to describe injecting a proportion "A" of the total current C driven between electrode E and electrode e. In this notation an indifferent electrode, the "earth" point of the system is defined as electrode 0. This could also be written [1,2,1]. A list of such definitions describes a complete distribution pattern. e.g. [1,0,1],[2,0,-1] describes a bipolar stimulation of between electrodes 1 and 2. Since scaled versions of the same stimulation pattern is applied to different electrodes, this definition is independent of the amplitude or waveform which can be biphasic, triphasic or any other waveshape. For both bipolar, and tripolar, the convention is that the second phase is cathodic for the stimulating electrode. This is done to put the nerve activation as late as possible.

Tripolar stimulation is described herein by the general definition [X,x,A],[Y,y,-1],[Z,z,1-A] where X,Y,Z are contact numbers, x,y,z are the nodes to which each of the other ends of the current sources are connected. In this document in most cases x=y=z=0 as it can be simpler to explain the situation of current sources between electrodes. Usually X <Y <Z indicating that the electrodes numbers are sequential and A <1. If A=1 or A=0 this becomes bipolar stimulation.

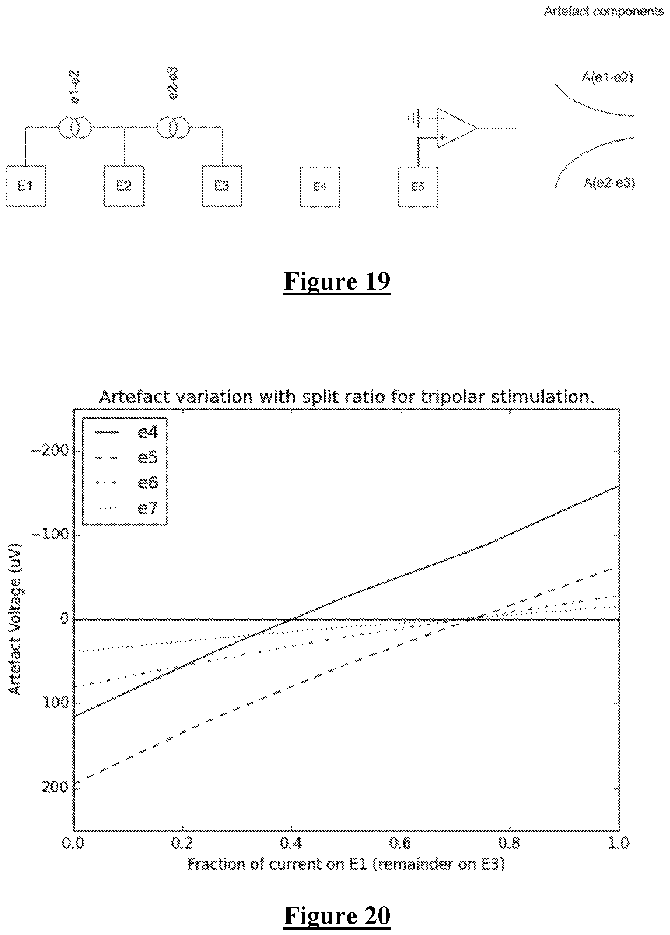

**Figure 18** shows that tripolar stimulation can be decomposed into two bipolar stimuli occurring simultaneously. **Figure 19** shows how the artefact from these two components will appear at the amplifier output. They will be of opposite phase and the artefact from the more distant electrode pair will be of lower amplitude. However, by adjusting the relative amplitude of the two bipolar stimuli, these components would be expected to cancel. Note in Fig. 18 that the central current source e2 could be omitted and replaced by a ground connection or by a switched connection to whichever voltage supply rail is appropriate for cathodic or anodic operation.

**Figure 20** shows simulated artefact for tripolar stimulation as the term A is varied - showing it has a null as expected. The null occurs for A approximately equal to 0.7. This illustrates that the method described in Fig. 19 works in principle. Figure 20 also shows that the ideal current ratio for creating an artefact null on E4 (being a ratio of about 0.4) is different to that from E5-E8, the latter all enjoying an artefact null at the same current ratio (about 0.7). Preferred embodiments would thus utilise differential neural measurements between two of E5-E8 so as to avoid E4, and allow artefact to be best minimised on both measurement electrodes simultaneously. This method of varying the current ratios in tripolar stimulation to achieve an artefact null represents one aspect of the present disclosure. Other examples may employ quadrupolar stimulation in order to steer an artefact minima to be co-located with a measurement electrode. Other examples might seek an observed minima in artefact which occurs when obtaining a differential measurement between two measurement electrodes, not when artefact on each electrode measurement electrode is zero or at a minima, but rather when the magnitude of artefact on each measurement electrode is the same, as occurs for example at a ratio of 0.2 if using E4 and E6 in Fig. 20.

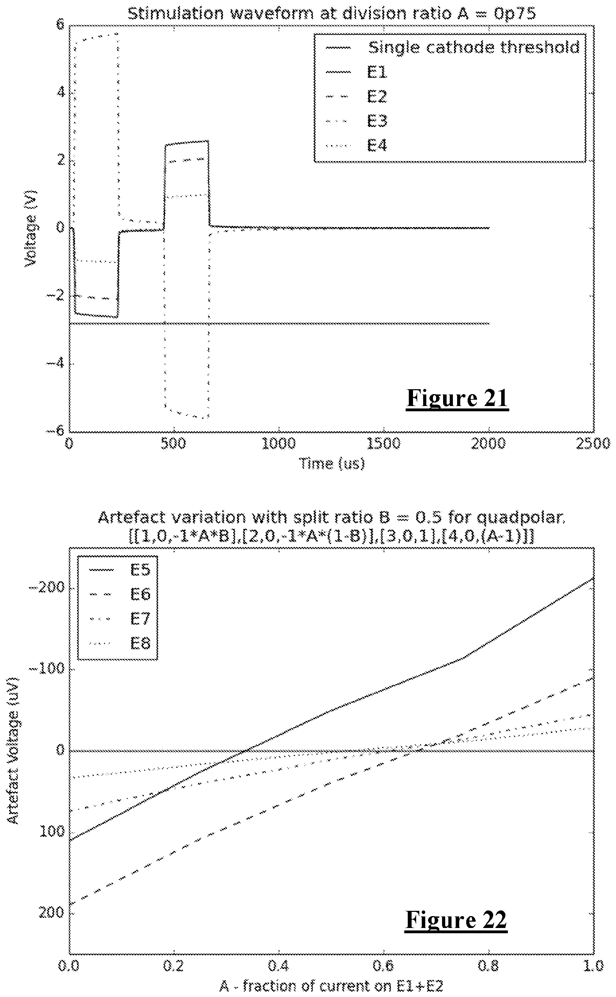

Quadrupolar can be described by the notation [1,0,A.B],[2,0,A.(1-B)],[3,0,-1],[4,0,1-A] where 0<A<1 and 0<B<1. "A" describes the charge division between the electrodes E1 and E2, and E3, which are proximal and distal to E3, while B defines the distribution of charge between E1 and E2. **Figure 21** shows a typical stimulation waveform for the case A=0.75, B=0.5. From this waveform it can be observed that the cathodic stimulation on E3 is more than twice the amplitude of the cathodic stimuli on the other electrodes. As a result E3 will be the only electrode producing an ECAP. It will also be observed that as there are three electrodes besides E3, and there are different combinations of charge distribution on the three electrodes where none of them have a current exceeding 50% of E3, and therefore there is an opportunity to adjust these ratios to create an artefact null while only generating a single ECAP. **Figure 22** shows the artefact amplitude on electrodes E4-E8 as the ratio A is varied. This shows there is an artefact null at A approximately equal to 0.7.

When A is equal to 0.7, and assuming the resistances of the electrodes are similar, the proportion of current in E1, E2 and E4 will be 35%, 35% and 30%. A similar effect would be expected when electrodes E1 E2 and E4 are simply connected together, which under the constant impedance approximation will result in division ratios of 33%, 33% and 33%. This is supported by clinical observations made by the present Applicant that in general "adding anodes reduces artefact". As the respective electrode impedances may not be the same, it may be that using individual current sources on each electrode provides a more robust result. Simulations indicate that the artefact reaches a null with a current division set to 37.5%, 37.5%, and 25%, with the null appearing at a similar ratio on E5 to E6 (E5 is the first available recording electrode in quadrupolar stimulation; E4 in tripolar stimulation). This quadrupolar approach thus represents a further aspect of the invention. It will be appreciated that this multipolar method can be extended as long as the number of return electrodes is greater than 2. For example, a stimulation profile of [1,0,A/2],[2,0,A/2],[3,0,-1],[4,0,(1-A)/2],[4,0,(1-A)/2] would be expected to produce an artefact null while maintaining conditions for a single cathode on E3.

Another example is described as a variant of tripolar stimulation [X,0,A],[Y,0,-1],[Z,0,1-A] where X,Y and Z are any three electrodes, where Z is used as a recording electrode, 0 < A < 1. Thus, in this embodiment artefact is nulled by injecting a small amount of current into a recording electrode, whereby artefact can thus be titrated.

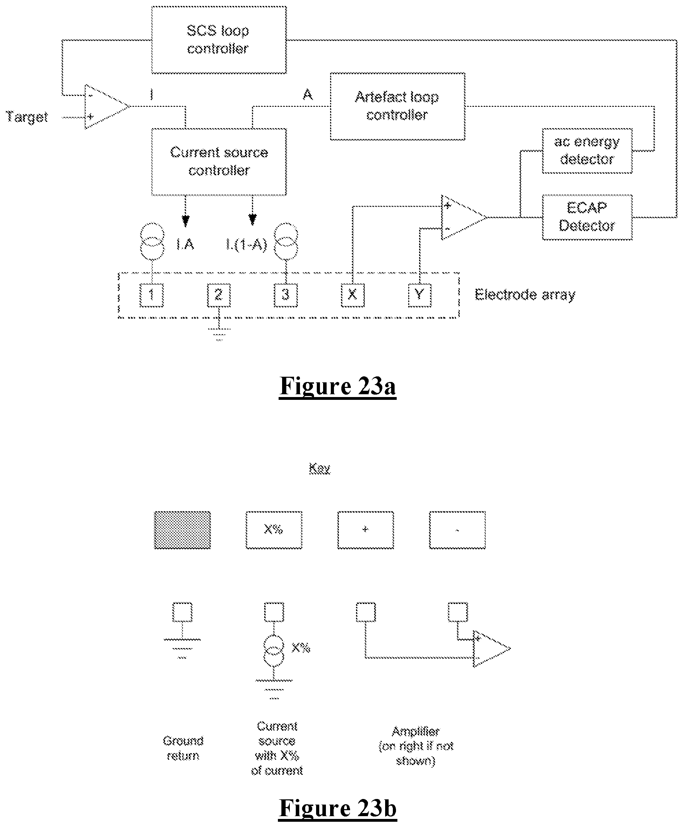

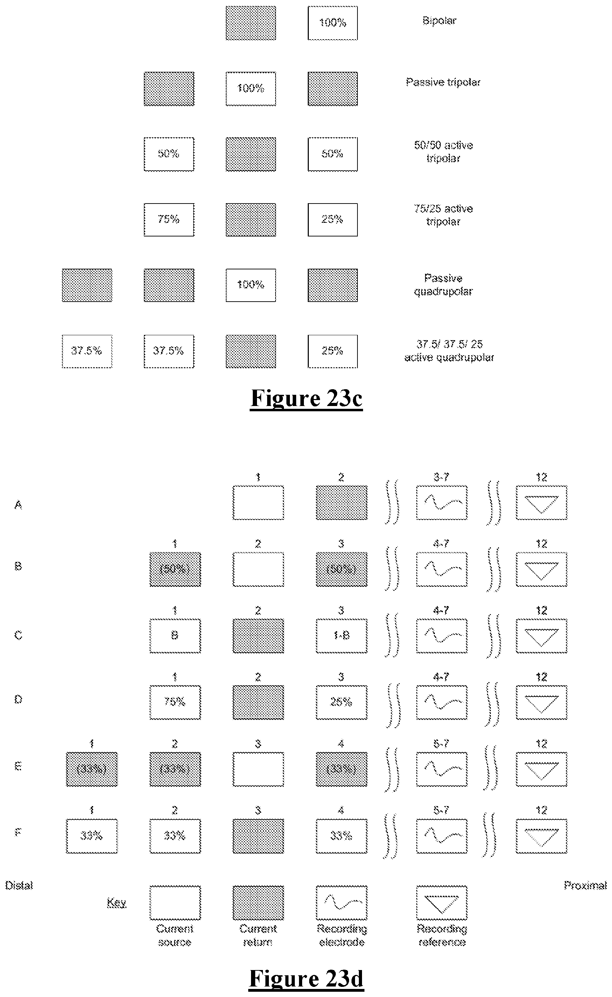

Another variant embodiment of tripolar stimulation may utilise automatic adjustment. In this embodiment the parameter A is adjusted automatically to minimize artefact, and it is embedded in a feedback loop to maintain an evoked response at a preset level. It is illustrated in **Figure 23****.** This embodiment has an ECAP feedback loop consisting of an ECAP detector and the SCS loop controller which generates a current value I. The present embodiment further provides for a second feedback loop configured to minimize artefact, this second loop comprising the ac energy detector, the artefact loop controller, and the current source controller. This second loop controls the parameter A that controls the division of current between electrodes 1 and 3.

The detector for the artefact loop measures the total energy on the recording electrodes. This energy will include both ECAP and artefact. However, the distribution of current between the cathodes will not affect the ECAP as the current remains constant. However, this variation in distribution will affect the artefact. At the point where the artefact is zero, only the ECAP will remain. The artefact nulling feedback loop operates by first measuring the total energy of the signal. This is best done by measuring the standard deviation of the signal samples over the measurement time. Such a measure is immune to the DC offset in the signal, based on the definition of standard deviation. The artefact loop controller then adjusts the control parameter A. Initially, this change can be in either direction. The artefact loop controller then measures the total energy a second time, to determine if it has increased or decreased. The artefact loop controller then changes the value A in the direction that decreases the detected energy.

In another embodiment, the architecture of Figure 23 may be adapted for sub-threshold determination of a preferred value for the parameter A. This embodiment provides an artefact minimisation algorithm which delivers a range of stimuli of varying stimulus ratio, at a sub-threshold level, being a stimulation level which is non-zero but which is small enough that it does not recruit any neural response. The artefact caused by each such sub-threshold stimulus at the measurement electrodes is then observed, in order to seek a stimulus ratio A which minimises artefact observed upon the measurement electrodes. This value of A is then used to configure a stimulus ratio of supra-threshold stimuli in ongoing therapeutic stimulation. The present inventors have found that a value for A which is optimised to minimise artefact at sub-threshold stimulus levels surprisingly is also well suited to minimising artefact at supra-threshold stimulation currents.

Options for current division are shown in **Figure 23c.** Figure 23c makes use of the symbol key shown in Figure 23b. The boxes are symbols for an electrode and its connection. A grey box indicates a current return (ground) connection for that electrode. A white box indicates a current source connection for that electrode, with the percentage of current for that electrode shown. Recording electrodes on the right are connected to the amplifier inputs. Recording electrodes are not shown in figure 23c for brevity.

As shown in Figure 23c, bipolar stimulation uses a single current source and a ground return. Tripolar stimulation uses two ground returns, usually on either side of the stimulating electrode. This is referred to in this document as "passive tripolar" to avoid ambiguity. In contrast, active tripolar connects the central contact to ground and connects current sources to the outer electrodes. 50/50 active tripolar is similar in behaviour to passive tripolar if the electrode impedances are equal, but the current division ratio will vary with tissue impedance, and thus over time as tissue grows around the stimulating electrode. Active tripolar is thus a preferred method to passive tripolar. Both passive tripolar and 50/50 active tripolar reduce artefact compared to bipolar stimulation, particularly for the adjacent electrode (as has been discussed herein). The present inventors have recognised that one particularly preferred method is to use active tripolar with a 75/25 current division ratio as this produces nulls at several of the recording contacts at the same time. Active tripolar or multipolar may in some embodiments even provide current sources for all stimulation electrodes, with no stimulus electrode being grounded, provided that the net cathodic current in each stimulus phase is balanced with the net anodic current in that phase. In such embodiments any mismatch in the current source matching may be addressed in accordance with the teachings of International Patent Publication No. WO2014071446.

A further method is to use passive quadrupolar. Assuming equal tissue impedance on all return electrodes, this achieves a 33/33/33 current division ratio, although it is noted that this simplistic assumption ignores the unequal distance of the respective grounded return electrodes which will tend to cause unequal return current division ratio. Nevertheless passive quadrupolar is preferred compared to passive tripolar as it produces lower artefact due to this difference in current division. Passive quadrupolar does however require room on the lead for an additional stimulating electrode. An active quadrupolar division ratio of 37.5/37.5/25 has similar artefact characteristics to 75/25 tripolar. There are many variants on these methods depending on the exact patient circumstances. However, the scope of the present invention is defined by the appended claims.

**Figure 23d** further illustrates multipolar stimulation configuration some of the measurement electrode configurations which might be used. Fig. 23c shows stimulation configurations for use with an implanted lead comprising 12 electrodes. While Fig. 23c shows electrodes 1-3 as being used for tripolar stimulation, and shows electrodes 1-4 being used for quadrupolar stimulation, other embodiments may deliver stimuli from any suitable subset of the 12 electrodes as described above in relation to Figs 1 and 2. In Fig. 23d, each row shows a different electrode stimulation configuration. Stimulation configuration B, for example, shows a tripolar stimulation configuration with a central stimulating electrode (2) and two lateral return electrodes (1 and 3) connected together. If tissue were homogenous, 50% of the current would return via each return, but because this varies with tissue impedance and may also change with posture the respective return currents cannot be assured. Thus, each return current value is shown in brackets, and the exact value of the respective return current through the return electrodes 1 and 3 (or electrodes 1, 2 and 4 in configuration E, for example) will depend on the patient anatomy. Once again, as shown in Row D, one particularly preferred method is to use active tripolar with a 75/25 current division ratio with the larger return current being forced away from the measurement electrodes. As shown in the stimulation configurations of Fig 23d, any other electrodes of the lead may be selected as the measurement electrodes, and any one of the three or four electrodes closest to the stimulus may be chosen as the sense electrode with the farthest electrode (12) being used as the measurement reference electrode..

It is to be noted that some of the stimulation configurations presented in Figure 23c, or more broadly throughout the other described embodiments, may not produce a zero-crossing in artefact. Additionally or alternatively, depending on a type of the measurement circuitry used, only a power output of the sensed signal may be retrieved. In such cases, a zero-crossing artefact may not exist or may not be detected. However, the present disclosure nevertheless recognise that a spatial minima in artefact may be produced and that steps may be taken in order for the minima to be "steered" or deliberately positioned upon or proximal to a measurement electrode.

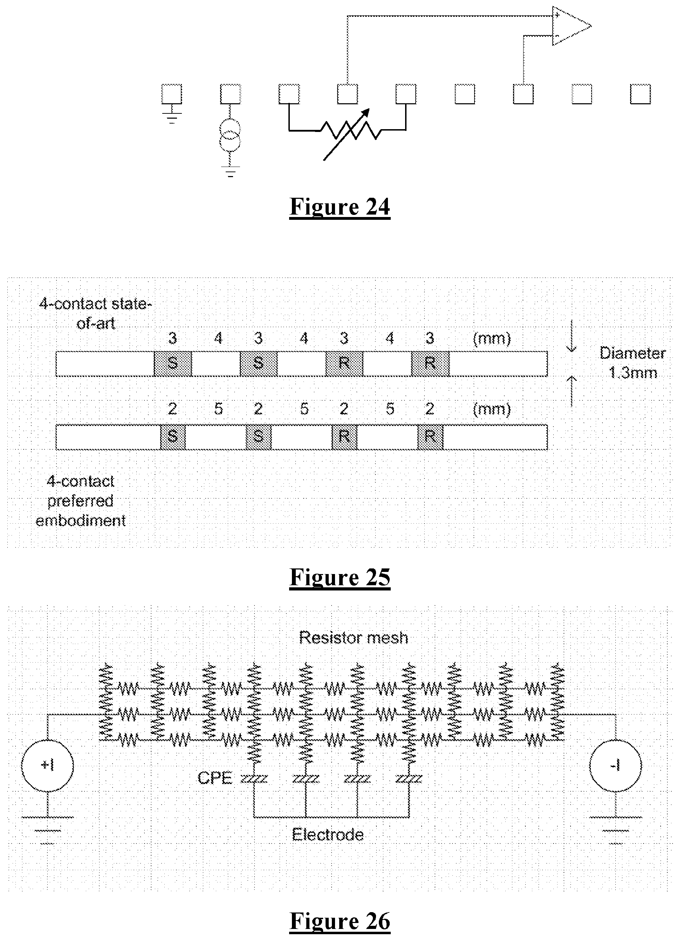

A further approach to reducing artefact is that a pair of electrodes not involved in the stimulation or measurement can be used to steer a zero onto a measurement electrode, as shown in **Figure 24****.**

In the example of Fig. 24, simply adding a resistance between electrodes 3 and 5 will permit artefact to be reduced close to zero on electrode 4 by adjustment of the joining resistance. Simulation predicts a value of 400 Ohms for the case above. Measurement is closer to 200 Ohms, which is quite good agreement. The resistance can be provided by switching an impedance realized by pulse-width modulating the connection. There may exist a number of such "bridge" connections that permit control of the artefact magnitude.

A third approach to artefact minimisation is to change the number, size and disposition of electrodes in a multi-electrode lead to minimize artefact on electrodes adjacent to stimulating electrodes.

Refer again to Figure 13. The graph was measured on standard leads with 3mm electrodes and 4mm insulating spaces for a 7mm period. Most simply, it is possible that a lead can be constructed with a small sensing electrode at the zero crossing, where minimum artefact will be measured. Simulation suggests that the 3rd and 4th electrodes are instrumental in creating the null, so they are left as before, and a small electrode is added at the correct position to sit in the zero between the 3rd and 4th electrodes. There will exist more than one pattern of electrodes and insulators that leaves an electrode at an artefact zero, and such variations are within the scope of the present invention.

A further solution offered involves a revised electrode design that, in a preferred embodiment, reduces artefact by 1/3 compared to current state of the art electrode designs. **Figure 25** in the upper portion shows a 4-contact version of a current state-of-the-art epidural electrode. Most such electrodes have 8 contacts, each being 3mm long and positioned apart on a 7mm pitch, as shown. In contrast, the preferred embodiment as shown in the lower portion of Figure 25 provides for 2mm long contacts on a 7mm pitch.

In Figure 25, some electrodes are marked 'S' to denote stimulation electrodes and some are marked 'R' denoting recording electrodes. It will be appreciated that the electrodes are substantially identical and any electrode can be used for stimulation and recording. However, this nomenclature is introduced at this point to support subsequent description. Figure 25 shows a situation where a 4-contact lead might be used for stimulation in a feedback SCS system, with two stimulating and two recording electrodes. In a system with 8 or 12 electrodes many more combinations of uses of the electrodes are available, as will be understood.

**Figure 26** shows the circuit model of a single electrode in a saline bath subject to a current that flows along the electrode. This would be rostro-caudally in a SCS situation. In this Figure the 3-dimensional structure of the saline bath is modeled as a 2-dimensional array of resistors and the electrode is modeled as a series of constant phase elements (CPEs), as described in the preceding. However, in Figure 26 the resistor mesh and CPE have been extended to show more resolution, and the electrode becomes a series of CPEs connected directly on the metallic side but distributed on the saline side. It has been found experimentally that 7 slices or more are required to adequately describe a 3mm electrode, though 4 are shown in this diagram for clarity, and this is a discrete model of a continuous situation and thus does not bear on the outcome. In this diagram the resistors at the perimeter are not connected. In practice this mesh will end at the limits of the physical volume it models.

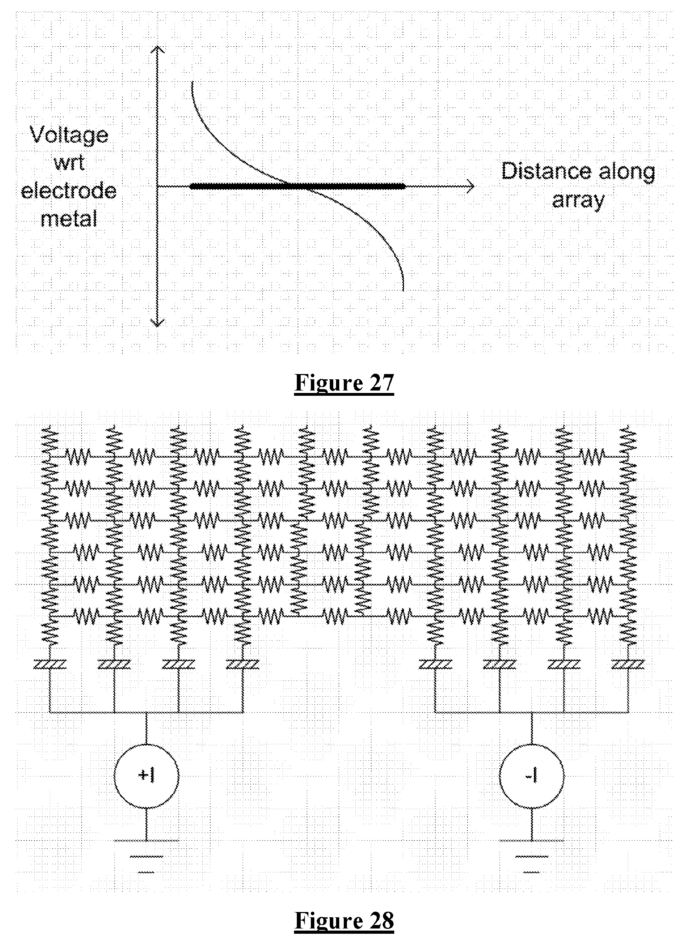

From this diagram it will be appreciated that if a voltage is applied between the right and left sides of the mesh, current will flow, and some will flow into the left-most CPE and then into the metallic electrode and this will then flow out of the right-most CPE. Current will also flow symmetrically, but to a lesser extent, through the centre two CPE elements. Consequently, at the end of some period, the voltage along the surface of the array, with respect to the metal, will be as shown in **Figure 27****.**

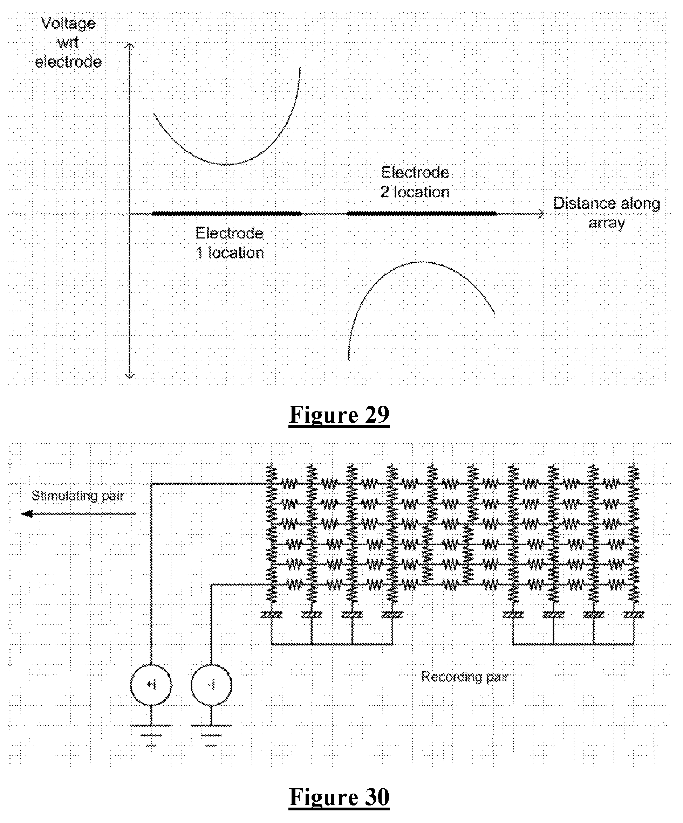

Now consider a pair of adjacent stimulating electrodes shown in **Figure 28****.** Current will flow preferentially where the contact metal is closest. After a period of stimulation, the voltage on the electrode surface relative to the metal will be as shown in **Figure 29****.**

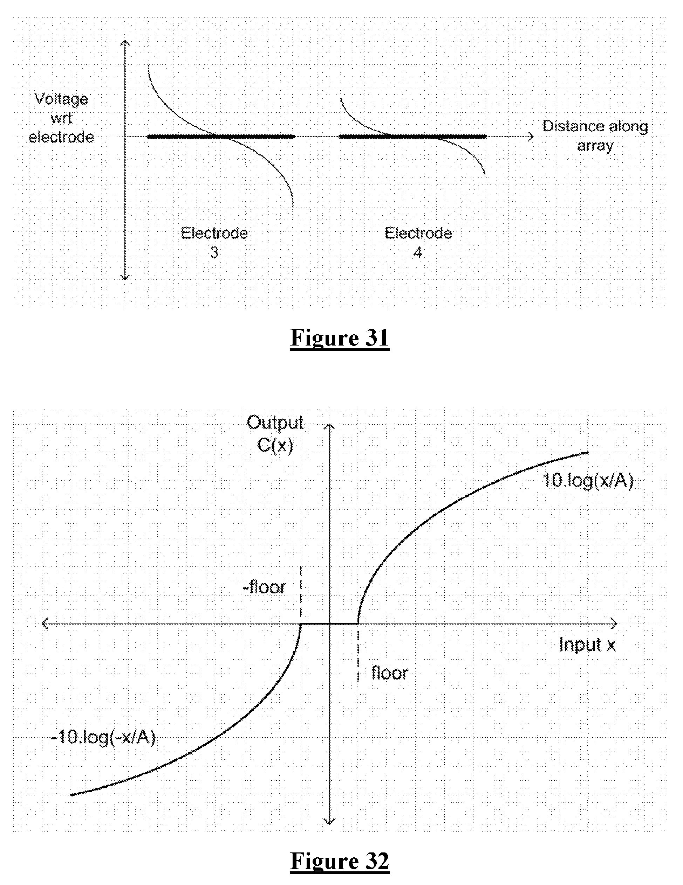

Now consider a pair of adjacent recording electrodes shown in **Figure 30****.** The stimulating pair, on the left but not shown, will create a vertical current gradient in the mesh. This will spread throughout the resistor mesh, affecting the closer recording electrode more than the more distant recording electrode. This current will be a fraction of the total stimulating current so is denoted with a small letter *i*. After a period of stimulation, the voltage on the recording electrodes' surface relative to the metal will be as in **Figure 31****.**

This again illustrates the main mechanisms of artefact generation: a voltage along a recording electrode which is greater for electrodes closer to the stimulation source and a gradient on the stimulating electrode which is largest on the edges where the stimulating contacts are close to one another. Artefact then occurs as this charge redistributes during the recording period, creating a changing differential voltage between the recording electrode metal contacts. The above-described simulations have shown that these effects are of comparable magnitude.

These phenomena are caused by dipoles created on the stimulating and recording electrodes. A dipole creates an electrical moment proportional to size and distance between the opposing charges. Thus reducing the length of the dipole will reduce the field and thus the artefact. As the dipole is made unbalanced by the proximity of the stimulating electrodes (as in Figure 29), increasing the gap between the electrodes is expected to lead to more symmetric charge distribution, and thus a smaller dipole. Simulations have shown that reducing the length of the electrode from 3mm to 2mm reduces the artefact by a factor of 3. This indicates that both mechanisms are present.

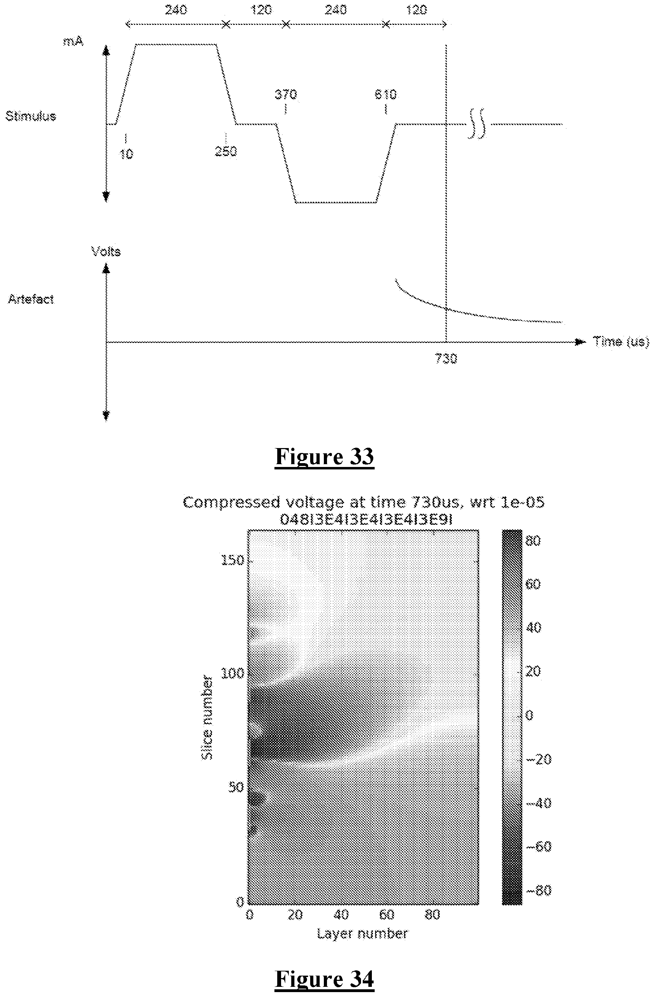

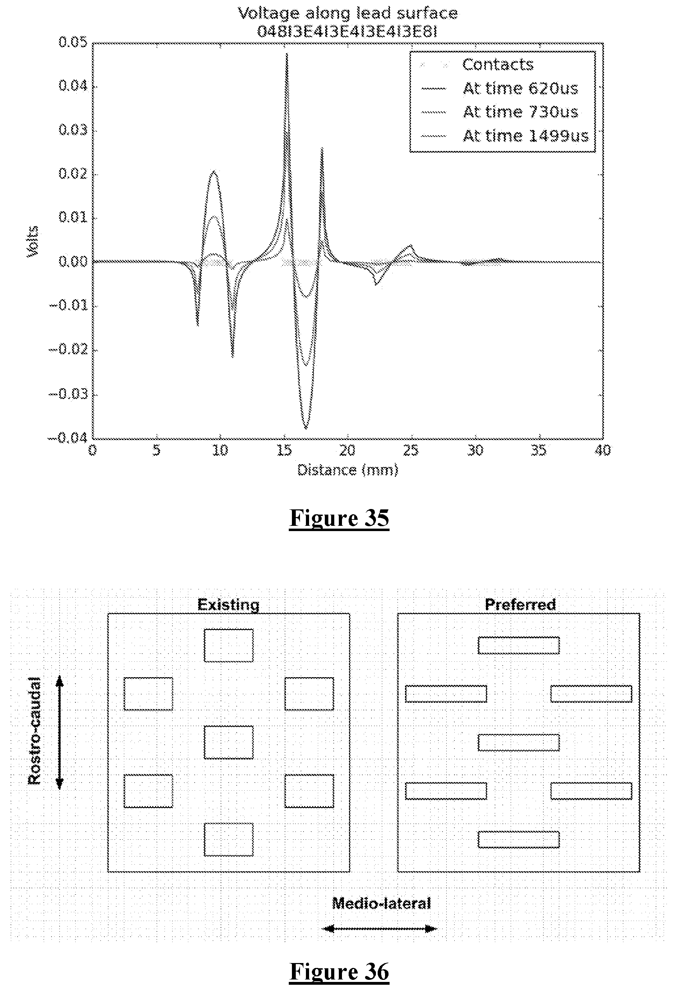

These phenomena can be seen in simulations of this shortened electrode configuration. The simulation was performed using the methods described in the preceding. **Figure 32** shows the compression algorithm used to highlight the phenomenon in a two-dimensional field plot. **Figure 33** shows the stimulation waveform, providing a reference for the subsequent graphs. **Figure 34** shows the compressed field in a saline bath at various times in response to a biphasic stimulation. **Figure 35** shows the uncompressed voltages along the electrode surface.

The system implications of reducing electrode size include the following. The impedance of an electrode varies with its area, and thus its length. Reducing an electrode length from 3mm to 2mm increases its impedance (as simulated in a saline bath with 1/10 saline in tripolar mode) from 750 to 950 ohms. This will increase the power required to drive the electrode and thus reduce battery life. However, this trade-off is acceptable and can be recovered by other system design changes such as improved current delivery mechanisms as per Australian Provisional Patent Application No. 2018900480.

Another system implication pertains to safe charge. The maximum charge that can be delivered through an electrode before unsafe radicals (such as Cl and H) are generated depends on the electrode area. A 3mm x 1.3mm electrode can be used to deliver 14.5uC. Since most patients require a charge less than 7uC to achieve comfort, and an electrode with a 2mm length can deliver 9.7uC before reaching its unsafe limit, an electrode length of 2 mm is thus more than adequate.

While Figures 25 to 35 are concerned with lead electrodes, paddle electrode contacts may also be made shorter in the rostral-caudal dimension in order to improve artefact suffered by such arrays. Figure 36 on the right side illustrates the shortening of contacts for this purpose in accordance with one example of the present disclosure.

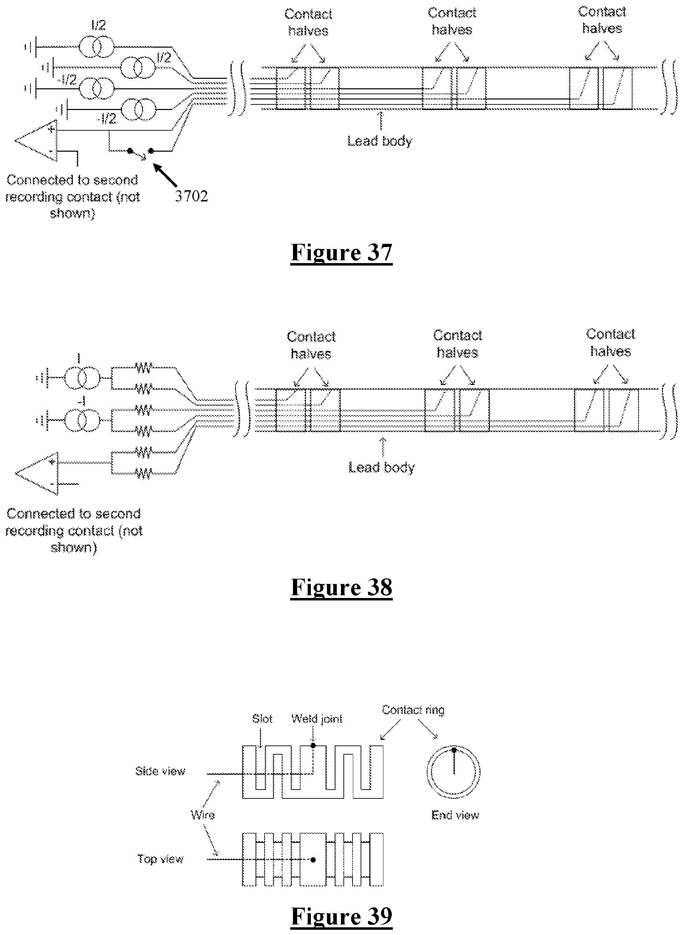

**Figure 37** illustrates yet another exemplary embodiment which exploits the preceding observations. In this embodiment each stimulating electrode is split into two rings that are independently driven. Splitting each electrode into two portions ensures that each portion of the electrode carries the same current, I/2. This arrangement will thereby counteract the asymmetrical voltages arising across a single electrode as shown in Figure 35. Similarly splitting the recording electrodes during stimulation blocks the current through the ring and thus counteracts the development of voltage asymmetries upon the recording electrode. The two portions of the recording electrode can be electrically connected together during the recording phase by means of the switch 3702 if required. The second recording electrode (not shown) would have a similar arrangement. While the embodiment of Figure 37 utilises two current sources for each stimulation electrode, with one current source being associated with each portion of an electrode, in another embodiment current flowing along the ring of the electrode can instead be attenuated by using split electrodes and adding resistance to the lead wire as shown in **Figure 38****.** This resistance can be added as individual components or by using connection wire with insulated strands, where each strand goes to a single ring.

A further variant is illustrated in **Figure 39****.** This embodiment involves adding resistance in a direction specific manner, whereby resistance is preferentially increased in a direction along the length of the contact using the contact material itself. This resistance inhibits the current flow and the development of the asymmetric voltage distributions associated with artefact as described in the preceding. In the embodiment of Figure 39, the additional longitudinal resistance is added by cutting slots into the ring. To achieve resistance comparable to the tissue impedance, the slots increase the current path length and thus the resistance. The ring is fed from a contact in the middle. To fabricate such slots might be performed using laser etching.

Some examples may utilise 3D printing for construction of the device. Accordingly, the present disclosure may reside in a digital blueprint comprising a digital file in a format configured for use with rapid prototyping and computer aided design (CAD) and/or manufacturing, such as being in the STL (stereolithography) file format. Such digital blueprint files, whether produced by performing a three dimensional scan of an embodiment of the invention, or produced by a CAD development software tool, or the like, are within the scope of the present invention.

Some examples of the present disclosure may be implemented in conjunction with other techniques of artefact minimisation or remediation, including for example the use of a triphasic stimulation technique in accordance with the teachings of the present Applicant's International Patent Publication No. WO2017219096.

It will be appreciated by persons skilled in the art that numerous variations and/or modifications may be made to the invention without departing from the scope of the invention as defined by the appended claims.

## Claims

1. A neurostimulation device comprising:
at least three stimulation electrodes configured to deliver an electrical stimulus in a multipolar fashion to neural tissue; and
at least one measurement electrode configured to record a response of the neural tissue to the stimulus,
wherein the stimulus and a position of the measurement electrode relative to the stimulus electrode are configured such that, in artefact as arising relative to distance from the stimulus electrode, a minima region of the artefact is substantially co-located with the measurement electrode by imposing a mismatch on the respective pairs of stimulation electrodes, in order to thereby co-locate the minima region of the artefact with the measurement electrode, and wherein the device is configured to adaptively alter a stimulation ratio, being a ratio between the respective portions of the stimulus current carried by the stimulus electrodes, depending on which measurement electrode(s) are in use.

2. The neurostimulation device of claim 1, further configured to carry out artefact minimisation by delivering a range of stimuli of varying stimulus ratio, at a sub-threshold level which does not recruit a neural response, observing the artefact caused by each such stimulus at the measurement electrodes, and seeking a stimulus ratio which minimises artefact observed upon the measurement electrodes in use,

3. The neurostimulation device of claim 1 or claim 2, further comprising an artefact minimisation feedback loop configured to adaptively alter the stimulation ratio.

4. The neurostimulation device of any one of claims 1 to 3, configured to deliver tripolar stimulus via three stimulus electrodes, comprising a central electrode which carries an entire stimulus current and two peripheral electrodes which carry two respective portions of the stimulus current to maintain charge-balanced stimulation, whereby the respective portions of the stimulus current carried by the peripheral stimulus electrodes are mismatched in a manner which causes the minima region of the artefact to be substantially co-located with the measurement electrode.

5. The neurostimulation device of any one of claims 1 to 3, configured to deliver a quadrupolar stimulation employing four stimulus electrodes.

6. The neurostimulation device of any one of claims 1 to 3, further comprising:
at least one passive electrode proximal to the measurement electrode; and
a variable impedance connected to the passive electrode, the impedance being configured to reduce artefact arising on the measurement electrode.

7. The neurostimulation device of claim 6 wherein the device is configured to adaptively control the variable impedance in order to reduce artefact observed on the at least one measurement electrode.

8. The neurostimulation device of claim 7 wherein the device is configured to deliver a range of stimuli while adjusting the variable impedance to take a range of distinct values, and observing the artefact caused by each such stimulus at the measurement electrode(s), in order to seek an impedance value which minimises artefact observed upon the measurement electrode(s) in use at the time.

9. The neurostimulation device of any one of claims 1 to 8 wherein artefact arising upon the measurement electrode is less than 75% of a peak artefact arising in spatial regions more distal from the stimulation electrode.

10. The neurostimulation device of claim 9 wherein artefact arising upon the measurement electrode is less than 50% of a peak artefact arising in spatial regions more distal from the stimulation electrode.

11. The neurostimulation device of claim 10 wherein artefact arising upon the measurement electrode is less than 25% of a peak artefact arising in spatial regions more distal from the stimulation electrode.

12. The neurostimulation device of any one of claims 1 to 11 wherein the minima region of artefact comprises a zero crossing region of artefact.

## Patentansprüche

1. Neurostimulationsvorrichtung, umfassend:
wenigstens drei Stimulationselektroden, die so konfiguriert sind, dass sie einen elektrischen Stimulus in multipolarer Weise an das Nervengewebe abgeben; und
wenigstens eine Messelektrode, die so konfiguriert ist, dass sie eine Antwort des Nervengewebes auf den Stimulus aufzeichnet,
wobei der Stimulus und eine Position der Messelektrode relativ zur Stimuluselektrode so konfiguriert sind, dass bei einem Artefakt, das relativ zum Abstand von der Stimuluselektrode entsteht, ein Minimalbereich des Artefakts im Wesentlichen mit der Messelektrode ko-loziert ist, indem den jeweiligen Paaren von Stimulationselektroden eine Fehlanpassung auferlegt wird, um dadurch den Minimalbereich des Artefakts mit der Messelektrode zu ko-lozieren, und wobei die Vorrichtung so konfiguriert ist, dass sie ein Stimulationsverhältnis, welches ein Verhältnis zwischen den jeweiligen Anteilen des von den Stimuluselektroden geführten Stimulationsstroms ist, adaptiv ändert, je nachdem, welche Messelektrode(n) verwendet wird/werden.

2. Neurostimulationsvorrichtung nach Anspruch 1, die ferner so konfiguriert ist, dass sie eine Artefaktminimierung durchführt, indem sie eine Reihe von Stimuli mit unterschiedlichem Stimulusverhältnis abgibt, mit einem unterschwelligen Pegel, der keine neuronale Antwort hervorruft, das durch einen jeden solchen Stimulus hervorgerufene Artefakt an den Messelektroden beobachtet, und ein Stimulusverhältnis sucht, das die an den verwendeten Messelektroden beobachteten Artefakte minimiert.

3. Neurostimulationsvorrichtung nach Anspruch 1 oder Anspruch 2, ferner umfassend eine Artefaktminimierungs-Rückkopplungsschleife, die so konfiguriert ist, dass sie das Stimulationsverhältnis adaptiv ändert.

4. Neurostimulationsvorrichtung nach einem der Ansprüche 1 bis 3, die so konfiguriert ist, dass sie einen tripolaren Stimulus über drei Stimuluselektroden abgibt, die eine zentrale Elektrode, die einen gesamten Stimulationsstrom führt, und zwei periphere Elektroden, die zwei jeweilige Anteile des Stimulationsstroms führen, um eine ladungsausgeglichene Stimulation aufrechtzuerhalten, umfassen, wobei die jeweiligen Anteile des Stimulationsstroms, die von den peripheren Stimuluselektroden geführt werden, in einer Weise fehlangepasst sind, die bewirkt, dass der Minimalbereich des Artefakts im Wesentlichen mit der Messelektrode ko-loziert ist.

5. Neurostimulationsvorrichtung nach einem der Ansprüche 1 bis 3, die so konfiguriert ist, dass sie eine quadrupolare Stimulation unter Verwendung von vier Stimuluselektroden liefert.

6. Neurostimulationsvorrichtung nach einem der Ansprüche 1 bis 3, ferner umfassend:
wenigstens eine passive Elektrode in der Nähe der Messelektrode; und
eine variable Impedanz, die mit der passiven Elektrode verbunden ist, wobei die Impedanz so konfiguriert ist, dass sie das an der Messelektrode auftretende Artefakt verringert.

7. Neurostimulationsvorrichtung nach Anspruch 6, wobei die Vorrichtung so konfiguriert ist, dass sie die variable Impedanz adaptiv steuert, um an der wenigstens einen Messelektrode beobachtete Artefakte zu verringern.

8. Neurostimulationsvorrichtung nach Anspruch 7, wobei die Vorrichtung so konfiguriert ist, dass sie eine Reihe von Stimuli abgibt, während sie die variable Impedanz so einstellt, dass sie einen Bereich verschiedener Werte annimmt, und das durch einen jeden solchen Stimulus hervorgerufene Artefakt an der/den Messelektrode(n) beobachtet, um einen Impedanzwert zu suchen, der das an der/den zu diesem Zeitpunkt verwendeten Messelektrode(n) beobachtete Artefakt minimiert.

9. Neurostimulationsvorrichtung nach einem der Ansprüche 1 bis 8, wobei das Artefakt, das an der Messelektrode entsteht, weniger als 75 % eines Spitzenartefakts beträgt, das in räumlichen Bereichen entsteht, die weiter von der Stimulationselektrode entfernt sind.

10. Neurostimulationsvorrichtung nach Anspruch 9, wobei das Artefakt, das an der Messelektrode entsteht, weniger als 50 % eines Spitzenartefakts beträgt, das in räumlichen Bereichen entsteht, die weiter von der Stimulationselektrode entfernt sind.

11. Neurostimulationsvorrichtung nach Anspruch 10, wobei das Artefakt, das an der Messelektrode entsteht, weniger als 25 % eines Spitzenartefakts beträgt, das in räumlichen Bereichen entsteht, die weiter von der Stimulationselektrode entfernt sind.

12. Neurostimulationsvorrichtung nach einem der Ansprüche 1 bis 11, wobei der Artefakt-Minimalbereich einen Artefakt-Nulldurchgangsbereich umfasst.

## Revendications

1. Dispositif de neurostimulation comprenant :
au moins trois électrodes de stimulation configurées pour délivrer un stimulus électrique de manière multipolaire à un tissu neural ; et
au moins une électrode de mesure configurée pour enregistrer une réponse du tissu neural au stimulus,
dans lequel le stimulus et une position de l'électrode de mesure par rapport à l'électrode de stimulation sont configurés de sorte que, dans un artefact apparaissant par rapport à la distance de l'électrode de stimulation, une région minimale de l'artefact est sensiblement colocalisée avec l'électrode de mesure en imposant un décalage sur les paires respectives d'électrodes de stimulation, afin de colocaliser ainsi la région minimale de l'artefact avec l'électrode de mesure, et le dispositif étant configuré pour modifier de manière adaptative un rapport de stimulation, qui est un rapport entre les parties respectives du courant de stimulation transporté par les électrodes de stimulation, en fonction de la ou des électrodes de mesure utilisées.

2. Dispositif de neurostimulation selon la revendication 1, configuré en outre pour effectuer une minimisation d'artefact en délivrant une gamme de stimuli de rapport de stimulation variable, à un niveau inférieur au seuil qui ne déclenche pas de réponse neurale, en observant l'artefact provoqué par chacun de tels stimuli au niveau des électrodes de mesure, et en recherchant un rapport de stimulation qui minimise l'artefact observé sur les électrodes de mesure utilisées.

3. Dispositif de neurostimulation selon la revendication 1 ou la revendication 2, comprenant en outre une boucle de rétroaction de minimisation d'artefact configurée pour modifier de manière adaptative le rapport de stimulation.

4. Dispositif de neurostimulation selon l'une quelconque des revendications 1 à 3, configuré pour délivrer un stimulus tripolaire via trois électrodes de stimulation, comprenant une électrode centrale qui transporte tout le courant de stimulation et deux électrodes périphériques qui transportent deux parties respectives du courant de stimulation afin de maintenir une stimulation à charge équilibrée, les parties respectives du courant de stimulation transportées par les électrodes de stimulation périphériques présentant un décalage de manière à ce que la région minimale de l'artefact soit sensiblement colocalisée avec l'électrode de mesure.

5. Dispositif de neurostimulation selon l'une quelconque des revendications 1 à 3, configuré pour délivrer une stimulation quadripolaire utilisant quatre électrodes de stimulation.

6. Dispositif de neurostimulation selon l'une quelconque des revendications 1 à 3, comprenant en outre :
au moins une électrode passive proximale à l'électrode de mesure ; et
une impédance variable reliée à l'électrode passive, l'impédance étant configurée pour réduire l'artefact apparaissant sur l'électrode de mesure.

7. Dispositif de neurostimulation selon la revendication 6, le dispositif étant configuré pour commander de manière adaptative l'impédance variable afin de réduire l'artefact observé sur l'au moins une électrode de mesure.

8. Dispositif de neurostimulation selon la revendication 7, le dispositif étant configuré pour délivrer une gamme de stimuli tout en réglant l'impédance variable afin qu'elle prenne une gamme de valeurs distinctes, et en observant l'artefact provoqué par chacun de tels stimuli au niveau de la ou des électrodes de mesure, afin de rechercher une valeur d'impédance qui minimise l'artefact observé sur la ou les électrodes de mesure utilisées à cet instant.

9. Dispositif de neurostimulation selon l'une quelconque des revendications 1 à 8, dans lequel l'artefact apparaissant sur l'électrode de mesure est inférieur à 75 % d'un artefact de crête apparaissant dans des régions spatiales plus distales de l'électrode de stimulation.

10. Dispositif de neurostimulation selon la revendication 9, dans lequel l'artefact apparaissant sur l'électrode de mesure est inférieur à 50 % d'un artefact de crête apparaissant dans des régions spatiales plus distales de l'électrode de stimulation.

11. Dispositif de neurostimulation selon la revendication 10, dans lequel l'artefact apparaissant sur l'électrode de mesure est inférieur à 25 % d'un artefact de crête apparaissant dans des régions spatiales plus distales de l'électrode de stimulation.

12. Dispositif de neurostimulation selon l'une quelconque des revendications 1 à 11, dans lequel la région minimale d'artefact comprend une région de passage à zéro d'artefact.
